# EUROPÄISCHE PATENTSCHRIFT

(11) **EP 3 157 913 B1**
(45) Veröffentlichungstag und Bekanntmachung des Hinweises auf die Patenterteilung: **17.04.2019**
(21) Anmeldenummer: 15728548.7
(22) Anmeldetag: 15.06.2015
(51) Int. Cl.: C07D 401/14, C07D 213/80, A61P 33/00, A01N 43/647

(54) **PYRAZOLYL-TRIAZOLYL-PYRIDINE ALS SCHÄDLINGSBEKÄMPFUNGSMITTEL**
PYRAZOLYL TRIAZOLYL PYRIDINES AS PESTICIDE
PYRAZOLYLE-TRIAZOLYLE-PYRIDINES EN TANT QUE PESTICIDE

(30) Priorität: 18.06.2014 EP 14172921
(43) Veröffentlichungstag der Anmeldung: 26.04.2017
(73) Patentinhaber: Bayer Animal Health GmbH, 40789 Monheim am Rhein (DE)
(72) Erfinder: SCHWARZ, Hans-Georg, 46282 Dorsten (DE); HALLENBACH, Werner, 40789 Monheim (DE); GÖRGENS, Ulrich, 40882 Ratingen (DE); ILG, Kerstin, 50670 Köln (DE); TURBERG, Andreas, 42781 Haan (DE)
(74) Vertreter: BIP Patents
(86) Internationale Anmeldenummer: PCT/EP2015/063271
(87) Internationale Veröffentlichungsnummer: WO 2015/193216

(56) Entgegenhaltungen:
- WO-A1-2010/130796
- WO-A1-2012/107434
- HOEKSTRA ET AL: "Potent, Orally Active GPIIb/IIIa Antagonists Containing a Nipecotic Acid Subunit. Structure-Activity Studies Leading to the Discovery of RWJ-53308", JOURNAL OF MEDICINAL CHEMISTRY, AMERICAN CHEMICAL SOCIETY, US, Bd. 42, Nr. 25, 1. Januar 1999 (1999-01-01), Seiten 5254-5265, XP002142349, ISSN: 0022-2623, DOI: 10.1021/JM990418B
- DATABASE REGISTRY [Online] CHEMICAL ABSTRACTS SERVICE, COLUMBUS, OHIO, US; 29. November 2011 (2011-11-29), XP002731144, Database accession no. 1346537-38-9
- DATABASE REGISTRY [Online] CHEMICAL ABSTRACTS SERVICE, COLUMBUS, OHIO, US; 29. November 2011 (2011-11-29), XP002731145, Database accession no. 1346533-38-7
- DATABASE REGISTRY [Online] CHEMICAL ABSTRACTS SERVICE, COLUMBUS, OHIO, US; 16. Dezember 2010 (2010-12-16), XP002731146, Database accession no. 1256794-68-9

## Beschreibung

Die vorliegende Anmeldung betrifft neue Pyrazolyl-triazolyl-pyridine, Verfahren zu ihrer Herstellung und ihre Verwendung zur Bekämpfung von tierischen Schädlingen, vor allem von Arthropoden und insbesondere von Insekten und Spinnentieren.

WO2012107434-A1 beschreibt bestimmte Pyrazolyl-triazolyl-pyridine als insektizide Verbindungen. Dabei umfasst die allgemeine Formel (A) in ihren Definitionen für A₁ bis A₄ CX (X steht für Wasserstoff, Halogen, Cyano, C₁-C₄-Alkyloxy, C₁-C₄-Alkyl oder C₁-C₄-Haloalkyl) oder Stickstoff, wobei Q² bestimmte Pyrazolyl-Substituenten bedeuten. Allerdings wird in den Vorzugsbereichen und in den Beispielen A₁, A₂ oder A₃ = Stickstoff nicht weiter ausgeführt.

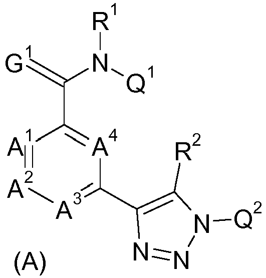

Moderne Pflanzenschutzmittel und veterinärmedizinische Ectoparasitizide müssen vielen Anforderungen genügen, beispielsweise in Bezug auf Dosierung, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln oder veterinärmedizinisch wirksamen Parasitiziden nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass Pyrazolyl-triazolyl-pyridine sowie deren N-Oxide und Salze dem Stand der Technik überlegene biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

### Zusammenfassung

Ein Aspekt der vorliegenden Erfindung bezieht sich auf Verbindungen der Formel (I) 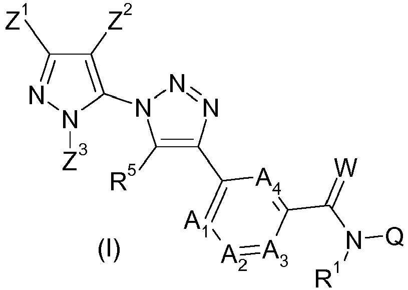 worin
- R¹: für Wasserstoff, jeweils gegebenenfalls substituiertes C₁-C₆-Alkyl, C₂-C₆-Alkenyl, C₂-C₆-Alkinyl, C₃-C₇-Cycloalkyl, C₃-C₇-Cycloalkyl-C₁-C₆-Alkyl, C₁-C₆-Alkylcarbonyl, C₁-C₆-Alkoxycarbonyl, C₁-C₆-Alkoxy-C₁-C₆-alkyl, Aryl-C₁-C₆-alkyl, Heteroaryl-C₁-C₆-alkyl steht;
die chemischen Gruppierungen
- A₁: für CR² oder Stickstoff,
- A₂: für Stickstoff,
- A₃: für CR³ oder Stickstoff und
- A₄: für CR⁴ oder Stickstoff stehen,
wobei aber nicht mehr als drei der chemischen Gruppierungen A₁ bis A₄ gleichzeitig für Stickstoff stehen;
- R², R³ und R⁴: unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N-*Di-(C₁-C₆-Alkyl)amino, C₁-C₆-Alkylsulfonyl-amino, oder *N*-(C₁-C₆-Alkyl)-C₁-C₆-alkylsulfonyl-amino stehen;
- W: für Sauerstoff oder Schwefel steht;
- Q: für Wasserstoff, Formyl, Hydroxy, Amino oder eine der jeweils gegebenenfalls substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
- Q: für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
- V: für Halogen, Cyano, Nitro, oder eine der jeweils gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-Alkyl, C₁-C₆-Alkylsulfanyl, C₁-C₆-Alkylsulfinyl, C₁-C₆-Alkylsulfonyl, *N*-(C₁-C₆-Alkyl)amino, oder *N,N*-Di(C₁-C₆-Alkyl)amino steht;
- R⁵: unabhängig voneinander für Wasserstoff, Halogen, Cyano, Nitro, Amino, jeweils gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆)-Alkylamino oder *N,N-*Di-(C₁-C₆-Alkyl)amino steht;
- Z¹: für ein gegebenenfalls substituiertes C₁-C₆-Alkyl steht;
- Z²: für Wasserstoff, Halogen, Cyano, Nitro oder jeweils gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl steht; und
- Z³: für Wasserstoff oder jeweils gegebenenfalls substituiertes C₁-C₆-Alkyl, C₃-C₇-Cycloalkyl, C₃-C₆-Alkenyl, C₃-C₆-Alkinyl, Aryl oder Hetaryl steht, wobei "gegebenenfalls substituiert", soweit keine spezifischen Substituenten angegeben sind, heißt, dass die entsprechende Gruppe einfach oder mehrfach mit einem Substituenten M¹ substituiert sein kann, wobei bei Mehrfachsubstitutionen die Substituenten M¹ gleich oder verschieden sein können und die Substitutionen M¹ ausgewählt sind aus der Gruppe, die in Paragraph [0054] beschrieben ist.

Eine bevorzugte Ausführungsform bezieht sich auf eine erfindungsgemäße Verbindung der Formel (I), worin
- R¹: für Wasserstoff, oder jeweils gegebenenfalls ein- oder mehrfach unabhängig voneinander mit Halogen, Cyano, Alkoxy und Alkoxycarbonyl substituiertes C₁-C₆-Alkyl, C₂-C₆-Alkenyl, C₂-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, C₁-C₆-Alkoxy-C₁-C₆-alkyl, Aryl(C₁-C₃)-alkyl, Heteroaryl(C₁-C₃)-alkyl, steht;
die chemischen Gruppierungen
- A₁: für CR² oder Stickstoff,
- A₂: für Stickstoff,
- A₃: für CR³ oder Stickstoff, und
- A₄: für CR⁴ oder Stickstoff stehen,
wobei aber nicht mehr als drei der chemischen Gruppierungen A₁ bis A₄ gleichzeitig für Stickstoff stehen;
- R², R³ und R⁴: unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N*-Di-(C₁-C₆-alkyl)amino, C₁-C₆-Alkylsulfonylamino, oder *N*-(C₁-C₆-Alkyl)-C₁-C₆-alkylsulfonyl-amino stehen;
- W: für Sauerstoff oder Schwefel steht;
- Q: für Wasserstoff, Hydroxy, Formyl oder eine der jeweils gegebenenfalls unabhängig voneinander ein- oder mehrfach mit Hydroxy, Nitro, Amino, Halogen, Alkoxy, Cyano, Hydroxycarbonyl. Alkoxycarbonyl, Alkylcarbamoyl, Cycloalkylcarbamoyl oder Phenyl substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl, *N*-(C₁-C₆-Alkyl)amino, *N,N*-Di-(C₁-C₆-Alkyl)amino, oder *N*-(C₁-C₆-Alkylcarbonyl)amino steht; oder
- Q: für ein mit 0 - 4 Substituenten V substituierten Aryl oder für ein mit 0 - 4 Substituenten V substituierten 5 bzw. 6 gliedrigen Heteroaromaten steht, wobei
- V: unabhängig voneinander für Halogen, Cyano, Nitro, oder eine der jeweils gegebenenfalls unabhängig voneinander ein- oder mehrfach mit Hydroxy, Nitro, Amino, Halogen, Alkoxy, Cyano oder Phenyl substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di(C₁-C₆-Alkyl)amino steht;
- R⁵: unabhängig voneinander für Wasserstoff, Halogen, Cyano, Nitro, Amino oder jeweils gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di-(C₁-C₆-Alkyl)amino steht;
- Z¹: für ein jeweils gegebenenfalls mit Halogen substituiertes C₁-C₆-Alkyl steht;
- Z²: für Wasserstoff, Halogen, Cyano, Nitro, Amino oder ein jeweils gegebenenfalls mit Halogen substituiertes C₁-C₆-Alkyl, C₁-C₆-Alkylcarbonyl, C₁-C₆-Alkylsulfanyl, C₁-C₆-Alkylsulfinyl oder C₁-C₆-Alkylsulfonyl steht; und
- Z³: für Wasserstoff oder ein jeweils gegebenenfalls mit Halogen substituiertes C₁-C₆-Alkyl, C₃-C₆-Cycloalkyl, C₂-C₄-Alkenyl, C₂-C₄-Alkinyl, Aryl oder Hetaryl steht.

Eine weitere bevorzugte Ausführungsform bezieht sich auf eine erfindungsgemäße Verbindung der Formel (I), worin
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CR² oder Stickstoff,
- A₂: für Stickstoff,
- A₃: für CR³ oder Stickstoff, und
- A₄: für CR⁴ oder Stickstoff stehen,
wobei aber nicht mehr als drei der chemischen Gruppierungen A₁ bis A₄ gleichzeitig für Stickstoff stehen;
- R² und R⁴: unabhängig voneinander für Wasserstoff, Methyl, Fluor und Chlor stehen; und
- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
- W: für Sauerstoff oder Schwefel steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N*-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3 -yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
- R⁵: unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
- Z¹: für Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl steht;
- Z²: für Wasserstoff, Halogen, Cyano, Nitro,, Amino, Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl steht; und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl steht.

Eine weitere bevorzugte Ausführungsform bezieht sich auf eine erfindungsgemäße Verbindung der Formel (I), worin
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CH,
- A₂: für Stickstoff,
- A₃: für CR³, und
- A₄: für CH stehen;

- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
- W: für Sauerstoff steht; und
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N*-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3 -yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
- R⁵: für Wasserstoff, Methyl, Ethyl, 2-Methylethyl, tert.-Butyl, Fluor, Chlor, Brom, Iod, Nitro, Trifluormethyl, Amino steht;
- Z¹: für Trifluormethyl oder Pentafluorethyl steht;
- Z²: für Trifluormethyl, Difluormethyl, Nitro, Methylsulfanyl, Methylsulfinyl, Methylsulfonyl, Fluor, Chlor, Brom, Cyano oder Iod steht; und
- Z³: für Methyl, Ethyl, n-Propyl oder Wasserstoff steht.

Eine weitere bevorzugte Ausführungsform bezieht sich auf eine erfindungsgemäße Verbindung der Formel (I), wobei die Verbindung der Formel (I) eine Verbindung der Formel (Ia) darstellt 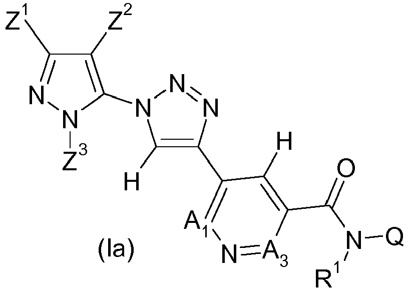 worin
- R¹: für Wasserstoff, Methyl oder Ethyl, bevorzugt Wasserstoff, steht; und
- A₁: für C-H oder C-(C₁-C₄-Alkyl) steht; bevorzugt für C-H steht; und
- A₃: für CR³ steht; worin
- R³: für Chlor, Fluor, Brom, Iod, C₁-C₄-Alkoxy oder Wasserstoff steht; bevorzugt für Chlor, Wasserstoff oder C₁-C₄-Alkoxy steht; mehr bevorzugt für Chlor, Wasserstoff oder Methoxy steht; und
- Z¹: für C₁-C₄-Alkyl oder C₁-C₄-Halogenalkyl steht; bevorzugt für Trifluormethyl oder Pentafluorethyl steht; ganz besonders bevorzugt für Pentafluoroethyl steht; und
- Z²: für C₁-C₄-Halogenalkyl, Nitro, C₁-C₄-Alkylsulfanyl, C₁-C₄-Alkylsulfinyl, C₁-C₄-Alkylsulfonyl, Fluor, Chlor, Brom, Cyano oder Iod steht, bevorzugt für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
- Z³: für Methyl, Ethyl, n-Propyl oder Wasserstoff steht; bevorzugt für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Eine weitere bevorzugte Ausführungsform bezieht sich auf eine erfindungsgemäße Verbindung der Formel (Ia) wobei eine Verbindung der Formel (Ia) eine Verbindung der Formel (Ib) darstellt worin 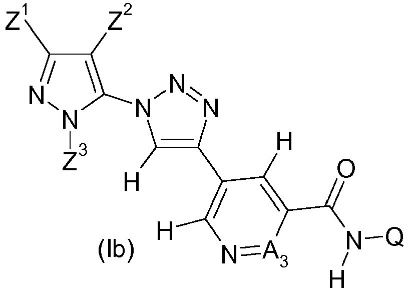
- A₃: für CR³ steht; worin
- R³: für Chlor, Wasserstoff oder C₁-C₄-Alkoxy steht; mehr bevorzugt für Chlor, Wasserstoff oder Methoxy steht; und
- Z¹: für Trifluormethyl oder Pentafluorethyl steht; ganz besonders bevorzugt für Pentafluoroethyl steht; und
- Z²: für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl, C₁-C₄-Halogenalkyl, C₁-C₄-Alkoxy oder C₁-C₄-Halogenalkoxy substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Eine weitere bevorzugte Ausführungsform bezieht sich auf eine erfindungsgemäße Verbindung der Formel (Ib), worin
- A₃: für CR³ steht; worin
- R³: für Chlor, Wasserstoff oder C₁-C₄-Alkoxy steht; mehr bevorzugt für Chlor, Wasserstoff oder Methoxy steht; und
- Z¹: für Pentafluoroethyl steht; und
- Z²: für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Eine weitere bevorzugte Ausführungsform bezieht sich auf eine Verbindung der Formel (Ib), worin
- A₃: für CR³ steht; worin
- R³: für Chlor, Wasserstoff oder C₁-C₄-Alkoxy steht und
- Z¹: für Pentafluoroethyl steht; und
- Z²: für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, substituiertes C₁-C₄-Alkyl, C₁-C₄-Halogenalkyl, C₃-C₆-Cycloalkyl, Phenyl, Benzyl, Pyridin, Pyrazol, Thiazol steht.

Eine weitere bevorzugte Ausführungsform bezieht sich auf eine Verbindung der Formel (Ib), worin
- A₃: für C-R³ steht; und
- A₃: für Chlor, Wasserstoff oder C₁-C₄-Alkoxy steht; und
- Z¹: für Pentafluoroethyl steht; und
- Z²: für Trifluomethyl steht; und
- Z³: für Methyl steht; und
- Q: für jeweils gegebenenfalls einfach mit Cyano substituiertes oder jeweils gegebenenfalls einfach, zweifach, dreifach vierfach oder fünffach unabhängig voneinander mit Fluor, Chlor, Brom, Iod, substituiertes C₁-C₄-Alkyl oder C₃-C₆-Cycloalkyl steht.

Ein weiterer Aspekt bezieht sich auf eine erfindungsgemäße Verbindung zur Verwendung zur Bekämpfung von Insekten, Spinnentieren und Nematoden.

Ein weiterer Aspekt bezieht sich auf eine pharmazeutische Zusammensetzung, enthaltend wenigstens eine erfindungsgemäße Verbindung.

Ein weiterer Aspekt bezieht sich auf eine erfindungsgemäße Verbindung zur Verwendung als Arzneimittel.

Ein weiterer Aspekt bezieht sich auf die Verwendung einer erfindungsgemäßen Verbindung zur Herstellung pharmazeutischer Zusammensetzungen zur Bekämpfung von Parasiten auf Tieren.

Ein weiterer Aspekt bezieht sich auf ein Verfahren zur Herstellung von Pflanzenschutzmitteln enthaltend mindestens eine erfindungsgemäße Verbindung, sowie übliche Streckmittel und/oder oberflächenaktive Substanzen.

Beschrieben wir auch eine Verbindung der Formel (III) 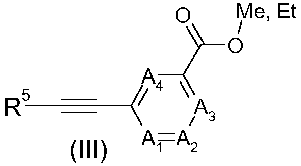 worin O-Me für -O-CH₃ steht und -O-Et für -O-CH₂-CH₃ steht, die chemischen Gruppierungen
- A₁: für CH,
- A₂: für Stickstoff,
- A₃: für CR³, und
- A₄: für CH stehen;
- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht; und
- R⁵: für Wasserstoff, Methyl, Ethyl, 2-Methylethyl, tert.-Butyl, Trifluormethyl steht.

Ein weiterer Aspekt bezieht sich auf die Verwendung einer Verbindung der Formel (III) zur Herstellung einer Verbindung der Formel (I).

Ein weiterer Aspekt bezieht sich auf die Verwendung der erfindungsgemäßen Verbindungen zum Schutz des Vermehrungsmaterials von Pflanzen, bevorzugt zum Schutz von Saatgut.

### Definitionen

Der Fachmann ist sich bewusst, dass die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Der Ausdruck "gegebenenfalls substituiert", soweit keine spezifischen Substituenten angegeben sind, heißt, dass die entsprechende Gruppe einfach oder mehrfach mit einem Substituenten M¹ substituiert sein kann, wobei bei Mehrfachsubstitutionen die Substituenten M¹ gleich oder verschieden sein können.

Dem Fachmann ist klar, dass in dieser Anmeldung genannte Beispiele nicht als beschränkend anzusehen sind sondern lediglich einige Ausführungsformen näher beschreiben.

Bei den in den vorstehenden Formeln angegebenen Definitionen der Symbole wurden Sammelbegriffe verwendet, die allgemein repräsentativ für die folgenden Substituenten stehen:

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen besonders bevorzugt mit 1, 2, 3 oder 4 Kohlenstoffatomen, wie beispielsweise Methyl, Ethyl, n-Propyl, Isopropyl, n-Butyl, Isobutyl, s-Butyl, t-Butyl Die erfindungsgemäßen Alkyle können mit einem oder mehreren, gleichen oder verschiedenen Resten M¹ gegebenenfalls substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen besonders bevorzugt mit 2, 3 oder 4 Kohlenstoffatomen, und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, etc. Die erfindungsgemäßen Alkenyle können mit einem oder mehreren, gleichen oder verschiedenen Resten M¹ gegebenenfalls substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen besonders bevorzugt mit 2, 3 oder 4 Kohlenstoffatomen, und mindestens einer Dreifachbindung wie beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, etc.. Die erfindungsgemäßen Alkinyle können mit einem oder mehreren, gleichen oder verschiedenen Resten M¹ gegebenenfalls substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Bicyclo[2.2.1]heptyl, Bicyclo[2.2.2]octyl oder Adamantyl, besonders bevorzugt für Cycloalkyle mit 3, 4, 5, 6 oder 7 Kohlenstoffatomen, wie z. B. Cyclopropyl oder Cyclobutyl. Die erfindungsgemäßen Cycloalkyle können mit einem oder mehreren, gleichen oder verschiedenen Resten M¹ gegebenenfalls substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, Besonders brzugt für Alkylcycloalkyle mit 4, 5 oder 7 Kohlenstoffatomen wie z. B. Ethylcyclopropyl oder 4-Methyl-cyclohexyl, wobei das Alkylcycloalkyl über das Cycloalkyl mit der Grundstruktur verbunden ist. Die erfindungsgemäßen Alkylcycloalkyle können mit einem oder mehreren, gleichen oder verschiedenen Resten M¹ gegebenenfalls substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, besonders bevorzugt für Cycloalkylalkyle mit 4, 5 oder 7 Kohlenstoffatomen wie unter anderen Cyclopropylmethyl oder Cyclobutylmethyl, wobei das Cycloalkylalkyl über das Alkyl mit der Grundstruktur verbunden ist. Die erfindungsgemäßen Cycloalkylalkyle können mit einem oder mehreren, gleichen oder verschiedenen Resten M¹ gegebenenfalls substituiert sein.

Erfindungsgemäß steht "Alkoxy" für geradkettiges oder verzweigtes O-Alkyl, vorzugsweise mit 1 bis 6 Kohlenstoffatomen, mehr bevorzugt für Alkoxygruppen mit 1 bis 4 Kohlenstoffatomen wie beispielsweise Methoxy, Ethoxy, n-Propoxy, Isopropoxy, n-Butoxy, Isobutoxy, s-Butoxy oder t-Butoxy. Die erfindungsgemäßen Alkoxygruppen können mit einem oder mehreren, gleichen oder verschiedenen Resten M¹ gegebenenfalls substituiert sein.

Erfindungsgemäß steht "Alkylsulfanyl" für geradkettiges oder verzweigtes S-Alkyl, vorzugsweise mit 1 bis 6 Kohlenstoffatomen, mehr bevorzugt für Alkylsulfanylgruppen mit 1 bis 4 Kohlenstoffatomen, wie beispielsweise Methylthio, Ethylthio, n-Propylthio, Isopropylthio, n-Butylthio, Isobutylthio, s-Butylthio und t-Butylthio. Die erfindungsgemäßen Alkylsulfanylgruppen können mit einem oder mehreren, gleichen oder verschiedenen Resten M¹ gegebenenfalls substituiert sein.

Erfindungsgemäß steht "Alkylsulfinyl" für geradkettiges oder verzweigtes Alkylsulfinyl, vorzugsweise mit 1 bis 6 Kohlenstoffatomen, mehr bevorzugt für Alkylsulfinylgruppen mit 1 bis 4 Kohlenstoffatomen, wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Die erfindungsgemäßen Alkylsulfinylgruppen können mit einem oder mehreren, gleichen oder verschiedenen Resten M¹ gegebenenfalls substituiertsein.

Erfindungsgemäß steht "Alkylsulfonyl" für geradkettiges oder verzweigtes Alkylsulfonyl, vorzugsweise mit 1 bis 6 Kohlenstoffatomen, mehr bevorzugt für Alkylsulfonylgruppen mit 1 bis 4 Kohlenstoffatomen, wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Die erfindungsgemäßen Alkylsulfonylgruppen können mit einem oder mehreren, gleichen oder verschiedenen Resten M¹ gegebenenfalls substituiert sein.

Erfindungsgemäß steht "Acyl" für Reste, die eine X¹-C(=O)-X² Gruppe enthalten, wobei X¹ und X² unabhängig voneinander für einen organischen Rest wie in dieser Anmeldung definiert oder für Wasserstoff oder für eine Bindung an die Grundstruktur einer Verbindung der Formel (I) steht. Insbesondere werden unter "Acyl" organische Säuren, Ester, Aldehyde, Alkylcarbonyl (alkyl-C(=O)-) und Amide verstanden. Bevorzugt handelt es sich bei X¹ und X² jeweils unabhängig voneinander um eine gegebenenfalls mit einem oder mehreren, gleichen oder verschiedenen Resten M¹ substituierte Gruppe ausgewählt aus Alkyl, Alkylen (-CₙH₂ₙ-), Alkoxy, Alkoxylen (-O-CₙH2ₙ-), Amino, Mono- oder Di-Alkylamino oder Wasserstoff oder ein Rest X¹ oder X² stellt eine Bindung an die Grundstruktur einer Verbindung der Formel (I) dar.

Erfindungsgemäß steht "Alkylcarbonyl" für geradkettiges oder verzweigtes Alkyl-C(=O)-, vorzugsweise mit 2 bis 7 Kohlenstoffatomen (inklusive des C-Atoms der C(=O)-Gruppe), mehr bevorzugt für Alkylcarbonyle mit 2 bis 5 Kohlenstoffatomen ((C₁-C₄)Alkyl-C(=O)-), wie Methylcarbonyl, Ethylcarbonyl, n-Propylcarbonyl, Isopropylcarbonyl, s-Butylcarbonyl und t-Butylcarbonyl. Die erfindungsgemäßen Alkylcarbonyle können mit einem oder mehreren, gleichen oder verschiedenen Resten M¹ gegebenenfalls substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, mehr bevorzugt für Cycloalkylcarbonyl mit 3, 5 oder 7 Kohlenstoffatomen im Cycloalkylteil wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Die erfindungsgemäßen Cycloalkylcarbonylgruppen können mit einem oder mehreren, gleichen oder verschiedenen Resten M¹ gegebenenfalls substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen, mehr bevorzugt mit 1, 2, 3 oder 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten M¹ gegebenenfalls substituiert sein.

Erfindungsgemäß steht "Halogen" für Fluor (F), Chlor (Cl), Brom (Br) oder Iod (I).

Die Ausdrücke "Halogenalkyl", "Halogenalkenyl", "Halogenalkinyl", "Halogenalkylcarbonyl" "Halogenalkoxy", "Halogenalkoxycarbonyl", "Halogenalkylsulfanyl", "Halogenalkylsulfinyl" oder "Halogenalkylsulfonyl" wie hierin benutzt beziehen sich auf mit mindestens einem Halogen substituierte chemische Alkyl, Alkenyl, Alkinyl, Alkylcarbonyl, Alkoxy, Alkoxycarbonyl, Alkylsulfanyl, Alkylsulfinyl oder Alkylsulfonyl Gruppe (jeweils bevorzugt mit eins bis 6 Kohlenstoffatomen oder mehr bevorzugt mit eins, zwei, drei oder vier Kohlenstoffatomen). Die Halogengruppen können einfach oder mehrfach bis zur maximal möglichen Substituentenzahl (perhalogeniert) mit Halogen substituiert sein. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor oder Chlor und besonders bevorzugt für Fluor. In einer bevorzugten Ausführungsform sind perhalogenierte Gruppen mit nur einer Sorte Halogenen maximal substituiert, z. B. perfluoriertes Methyl (Trifluormethyl; CF₃) oder perfluoriertes Ethyl (Pentafluorethyl; C₂F₅). Einige Beispiele für "Halogenalkyl", "Halogenalkenyl", "Halogenalkinyl", "Halogenalkylcarbonyl" "Halogenalkoxy", "Halogenalkoxycarbonyl", "Halogenalkylsulfanyl", "Halogenalkylsulfinyl" oder "Halogenalkylsulfonyl" sind Trichlormethyl (CCl₃) , Trifluormethyl (CF₃), Chlordifluormethyl (CClF₂), Dichlorfluormethyl (CCl₂F), 2,2-Difluorethyl (F₂HCCH₂), 2,2,2-Trifluorethyl (F₃CCH₂), Pentafluorethyl (C₂F₅), 2,2-Difluorethenyl (CHCF₂), 2-Chlorethinyl (CHCCl), Trifluormethoxy -OCF₃, Difluormethoxy -OCHF₂, 1,1,2,2-Tetrafluorethylthio, 2-Chlor-1,1,2-trifluorethylsulfinyl, Trichlormethylsulfonyl, etc. Die erfindungsgemäßen Halogengruppen können mit einem oder mehreren, gleichen oder verschiedenen Resten M¹ gegebenenfalls substituiert sein, solange wenigstens ein Wasserstoffatom an einem Kohlenstoffatom der Halogengruppe durch ein Halogen ersetzt ist. Ein Beispiel für ein mit einem M¹ substituierten Halogenalkyl ist 2-Cyano-2,2-Difluoroethyl (C(CN)F₂CH₂).

Eine Aminogruppe (-NH₂) kann mit mit einem oder mehreren, gleichen oder verschiedenen Resten M¹ gegebenenfalls substituiert sein.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N*-Diethylamino, *N,N*-Di-n-propylamino, *N,N*-Diisopropylamino oder *N,N*-Dibutylamino), *N*-Mono-oder *N,N*-Dialkoxyalkylaminogruppen (z.B. *N*-Methoxymethylamino, *N*-Methoxyethylamino, *N,N*-Di-(methoxymethyl)-amino oder *N,N*-Di-(methoxyethyl)-amino), *N*-Mono- und *N,N*-Diarylamino, wie gegebenenfalls substituierte Aniline, Acylamino, *N,N*-diacylamino, *N*-Alkyl-*N*-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder gegebenenfalls substituiertes Phenyl; für Acyl gilt dabei die weiter oben genannte Definition, vorzugsweise (C₁-C₄)Alkyl-C(=O)-.

Substituiertes Amino schließt auch quartäre Ammoniumverbindungen (Salze) mit vier organischen Substituenten am Stickstoffatom ein.

Erfindungsgemäß steht "Hydroxyalkyl" für geradkettigen oder verzweigten Alkohol, vorzugsweise mit 1 bis 6 Kohlenstoffatomen, mehr bevorzugt mit 1, 2, 3 oder 4 Kohlenstoffatomen, wie beispielsweise Methanol, Ethanol, n-Propanol, Isopropanol, n-Butanol, Isobutanol, s-Butanol und t-Butanol. Die erfindungsgemäßen Hydroxyalkylgruppen können mit einem oder mehreren, gleichen oder verschiedenen Resten M¹ substituiert sein

Erfindungsgemäß steht "Alkylaminocarbonyl" für geradkettiges oder verzweigtes Alkylaminocarbonyl mit vorzugsweise 1 bis 6 Kohlenstoffatomen, mehr bevorzugt 1, 2, 3 oder 4 Kohlenstoffatomen im Alkylteil, wie beispielsweise Methylaminocarbonyl (-CONHCH₃), Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten M¹ gegebenenfalls substituiert sein.

Erfindungsgemäß steht "*N,N*-Dialkylamino-carbonyl" (-C(=O)N(alkyl)₂)für geradkettiges oder verzweigtes *N,N*-Dialkylaminocarbonyl mit vorzugsweise 1 bis 6 Kohlenstoffatomen pro alkyl, mehr bevorzugt 1, 2, 3, oder 4 Kohlenstoffatomen pro alkyl, wie beispielsweise *N,N*-Dimethylamino-carbonyl (-C(=O)N(CH₃)₂), N.N-Diethylamino-carbonyl, *N,N*-Di(n-propylamino)-carbonyl, *N,N*-Di-(isopropylamino)-carbonyl und *N,N*-Di-(s-butylamino)-carbonyl. Die erfindungsgemäßen *N,N-*Dialkylamino-carbonylgruppen können mit einem oder mehreren, gleichen oder verschiedenen Resten M¹ gegebenenfalls substituiert sein.

"Carbozyklus", soweit nicht an anderer Stelle anders definiert ist insbesondere ein Cycloalkyl, Cycloalkenyl, oder Aryl. Insbesonder ist ein Carbozyklus ein C₆ bis C₁₄ mono- bi- oder tricyclisches Aryl. Ein Carbozyklus kann mit einem oder mehreren, gleichen oder verschiedenen Resten M¹ gegebenenfalls substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten M¹ gegebenenfalls substituiert sein.

Erfindungsgemäß steht "Arylalkyl" für ein mit einem Aryl substituierten Alkylrest mit vorzugsweise 6 bis 14, insbesondere 6 bis 10 Ring-Kohlenstoffatomen im Arylteil und 1 bis 6, insbesondere 1 bis 4 Kohlenstoffatomen im Alkylteil. Arylalkyl kann mit einem oder mehreren, gleichen oder verschiedenen Resten im Alkyl- und/oder Arylteil substituiert sein. Beispiele solcher Arylalkyle sind unter anderem Benzyl und 1-Phenylethyl. Die erfindungsgemäßen Arylalkylgruppen können mit einem oder mehreren, gleichen oder verschiedenen Resten M¹ gegebenenfalls substituiert sein.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl. Die erfindungsgemäßen Gruppen "Heterocyclus", "heterocyclischer Ring" oder "heterocyclisches Ringsystem" können mit einem oder mehreren, gleichen oder verschiedenen Resten M¹ gegebenenfalls substituiert sein.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten M¹ gegebenenfalls substituiert sein.

"Substituierte" Gruppe bzw. "mit mindestens einem Rest M¹ substituierten" Gruppe im Sinne der vorliegenden Erfindung, wie ein gegebenenfalls substituierter Alkyl-, Alkenyl-, Alkinyl-, Cycloalkyl-, Aryl-, Phenyl-, Benzyl-, Heterocyclyl-, Heteroaryl-, oder Amino-Rest etc., ist allgemein eine Gruppe die mindestens einen kohlenwasserstoffhaltigen oder stickstoffwasserstoffhaltigen Anteile enthält, in dem das Wasserstoff durch ein anderes Atom oder eine Atomgruppe M¹ ersetzt ist. In anderen Worten, eine derartige Gruppe ist eine vom unsubstituierten Grundkörper abgeleiteten substituierte Gruppe, wobei der Grundkörper mit einem oder mehreren Substituent(en) M¹, vorzugsweise 1, 2 oder 3 Resten M¹, substituiert ist und der/die Substituenten M¹ jeweils unabhängig voneinander ausgewählt aus der Gruppe bestehend aus Halogen, Hydroxy, Nitro, Formyl, Carboxy, Cyano, Amino, Isocyano, Azido, (C₁-C₄)-Alkyl, (C₁-C₄)-Halogenalkyl, (C₂-C₄)-Alkenyl, (C₂-C₄)-Alkinyl, (C₃-C₆)-Cycloalkyl, (C₁-C₄)-Alkoxy, (C₁-C₄)-Halogenalkoxy, (C₁-C₄)-Alkoxy-(C₁-C₄)-alkoxy, (C₁-C₄)-Alkoxy-(C₁-C₆)-alkyl, *N*-(C₁-C₄)-Alkoxy-imino-(C₁-C₃)-alkyl, (C₁-C₄)-Alkylsulfanyl, (C₁-C₄)-Halogenalkylsulfanyl, (C₁-C₄)-Alkoxycarbonyl, (C₁-C₄)-Alkylcarbonyl, Carbamoyl, C₁-C₄-Alkylcarbamoyl, C₃-C₇-Cycloalkylcarbamoyl, Mono- und *N,N*-Di(C₁-C₄)-alkylaminocarbonyl, Amino, (C₁-C₆)-Acylamino, Mono- und *N,N*-Di(C₁-C₄)-alkylamino, Tri(C₁-C₄)-alkylsilyl, (C₃-C₆)-Cycloalkyl, C₆-Aryl, Heterocyclyl mit 3 bis 6 Ringatomen, wobei jeder der letztgenannten cyclischen Gruppen auch über Heteroatome oder eine divalente funktionelle CH₂-, oder C₂H₄-Gruppe gebunden sein kann, (C₁-C₄)-Alkylsulfinyl, wobei beide Enantiomere der (C₁-C₄)-Alkylsulfinylgruppe umfasst sind, (C₁-C₄)-Alkylsulfonyl, (C₁-C₄)-Alkylphosphinyl, (C₁-C₄)-(C₁-C₄)-Alkylsulfanyl-(C₁-C₄)-alkyl, (C₁-C₄)-Alkoxy-(C₁-C₄)-alkyl, Mono- und *N,N*-Di(C₁-C₄)-alkyl-amino(C₁-C₄)-alkyl und Hydroxy(C₁-C₄)-alkyl ist/sind. Die beispielhaft genannten Reste M¹ können unsubstituiert sein oder gegebenenfalls (wie z. B. Alkyl oder Amino), sofern sie kohlenwasserstoffhaltige oder stickstoffwasserstoffhaltige Anteile enthalten, mit einen oder mehrere, vorzugsweise 1, 2 oder 3 Resten M² substituiert sein, wobei M² unabhängig voneinander ausgewählt ist aus der Gruppe bestehend aus Amino, Hydroxy, Halogen, Nitro, Cyano, Isocyano, Mercapto, Isothiocyanato, Carboxy und Carboamid.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Gegebenenfalls substituiertes Phenyl ist vorzugsweise Phenyl, das unsubstituiert oder ein- oder mehrfach, vorzugsweise einfach, zweifach oder dreifach durch gleiche oder verschiedene Reste aus der Gruppe Halogen, Cyano, Isocyano, Nitro, gegebenenfalls mit mindestens einem Rest M² substituiertes (C₁-C₄)Alkyl, C₂-C₄-Alkenyl, C₂-C₄-Alkinyl, (C₁-C₄)Alkoxy, (C₁-C₄)Alkoxy-(C₁-C₄)alkoxy, (C₁-C₄)Alkoxy-(C₁-C₄)alkyl, (C₃-C₆)-Cycloalkyl, (C₁-C₄)Halogenalkyl, (C₁-C₄)Halogenalkoxy, (C₁-C₄)Alkylsulfanyl, (C₁-C₄)Halogenalkylsulfanyl, substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Haloaklyl, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für mit Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Detaillierte Beschreibung

Die erfindungsgemäßen Pyrazolyl-triazolyl-pyridine sind durch die allgemeine Formel (I) 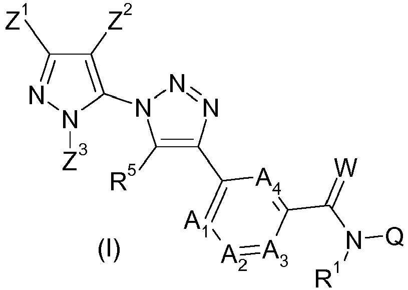 worin
- R¹: für Wasserstoff, jeweils gegebenenfalls substituiertes C₁-C₆-Alkyl, C₂-C₆-Alkenyl, C₂-C₆-Alkinyl, C₃-C₇-Cycloalkyl, C₃-C₇-Cycloalkyl-C₁-C₆-alkyl C₁-C₆-Alkylcarbonyl, C₁-C₆-Alkoxycarbonyl, C₁-C₆-Alkoxy-C₁-C₆-alkyl, Aryl-C₁-C₆-alkyl oder Heteroaryl-C₁-C₆-alkyl steht;
die chemischen Gruppierungen
- A₁: für CR² oder Stickstoff,
- A₂: für Stickstoff,
- A₃: für CR³ oder Stickstoff und
- A₄: für CR⁴ oder Stickstoff stehen,
wobei aber nicht mehr als drei der chemischen Gruppierungen A₁ bis A₄ gleichzeitig für Stickstoff stehen;
- R², R³ und R⁴: unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N*-Di-(C₁-C₆-Alkyl)amino, C₁-C₆-Alkylsulfonyl-amino, oder *N*-(C₁-C₆-Alkyl)-C₁-C₆-alkylsulfonyl-amino stehen;
- W: für Sauerstoff oder Schwefel steht;
- Q: für Wasserstoff, Formyl, Hydroxy, Amino oder eine der jeweils gegebenenfalls substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
- Q: für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
- V: für Halogen, Cyano, Nitro, oder eine der jeweils gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-Alkyl, C₁-C₆-Alkylsulfanyl, C₁-C₆-Alkylsulfinyl, C₁-C₆-Alkylsulfonyl, *N*-(C₁-C₆-Alkyl)amino, oder *N,N*-Di(C₁-C₆-Alkyl)amino steht;
- R⁵: unabhängig voneinander für Wasserstoff, Halogen, Cyano, Nitro, Amino, jeweils gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆)-Alkylamino oder *N,N*-Di-(C₁-C₆-Alkyl)amino steht;
- Z¹: für ein gegebenenfalls substituiertes C₁-C₆-Alkyl steht;
- Z²: für Wasserstoff, Halogen, Cyano, Nitro oder jeweils gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl steht; und
- Z³: für Wasserstoff oder jeweils gegebenenfalls substituiertes C₁-C₆-Alkyl, C₃-C₇-Cycloalkyl, C₃-C₆-Alkenyl, C₃-C₆-Alkinyl, Aryl oder Hetaryl steht, wobei "gegebenenfalls substituiert", soweit keine spezifischen Substituenten angegeben sind, heißt, dass die entsprechende Gruppe einfach oder mehrfach mit einem Substituenten M¹ substituiert sein kann, wobei bei Mehrfachsubstitutionen die Substituenten M¹ gleich oder verschieden sein können und die Substitutionen M¹ ausgewählt sind aus der Gruppe, die in Paragraph [0054] beschrieben ist.

Eine bevorzugte Ausführungsform bezieht sich auf eine erfindungsgemäße Verbindung der Formel (I), worin R¹ für Wasserstoff oder C₁-C₄-Alkyl steht, ganz besonders bevorzugt für Wasserstoff steht, und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0066] bis [0075] definiert sind.

Eine bevorzugte Ausführungsform bezieht sich auf eine erfindungsgemäße Verbindung der Formel (I), worin Z¹ und Z² unabhängig voneinander für perhalogeniertes (C₁-C₄)-Alkyl stehen (wobei Halogen ausgesucht ist aus Fluor, Chlor, Iod und Brom) und Z³ für (C₁-C₄)-Alkyl steht, ganz besonders bevorzugt Z¹ und Z² unabhängig voneinander für perfluoriertes (C₁-C₄)-Alkyl und Z³ für (C₁-C₄)-Alkyl steht (z. B. Z¹ steht für CF₃, Z² steht für C₂F₅ und Z³ steht für -CH₃) und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0066] bis [0075] definiert sind.

Eine bevorzugte Ausführungsform bezieht sich auf eine erfindungsgemäße Verbindung der Formel (I), worin A₃ für C-R³ steht und R³ und die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0066] bis [0075] definiert sind..

Eine besonders bevorzugte Ausführungsform bezieht sich auf eine erfindungsgemäße Verbindung der Formel (I), worin A₃ für C-R³ steht und R³ für Wasserstoff, Halogen ausgewählt aus Fluor, Chlor, Brom oder Iod, (C₁-C₄)-Alkyl oder (C₁-C₄)-Alkoxy steht (z. B. steht R³ bevorzugt für Wasserstoff, Fluor, Chlor, Brom, Iod, Methyl, Ethyl, Methoxy, Ethoxy, n-Propoxy, 1-Methylethoxy und ganz besonders bevorzugt für Methyl, Methoxy, Chlor oder Fluor) und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0066] bis [0075] definiert sind.

Eine bevorzugte Ausführungsform bezieht sich auf eine erfindungsgemäße Verbindung der Formel (I), worin W für Sauerstoff steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine bevorzugte Ausführungsform bezieht sich auf eine erfindungsgemäße Verbindung der Formel (I), worin A₄ und A₁ für -(C-H)- stehen und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine bevorzugte Ausführungsform bezieht sich auf eine erfindungsgemäße Verbindung der Formel (I), worin A₂ für Stickstoff steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine bevorzugte Ausführungsform bezieht sich auf eine erfindungsgemäße Verbindung der Formel (I), worin R⁵ für Wasserstoff steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine bevorzugte Ausführungsform bezieht sich auf eine erfindungsgemäße Verbindung der Formel (I), worin Q für jeweils gegebenenfalls einfach mit Cyano substituiertes oder jeweils gegebenenfalls einfach, zweifach, dreifach vierfach oder fünffach unabhängig voneinander mit Fluor, Chlor, Brom, Iod, substituiertes C₁-C₄-Alkyl oder C₃-C₆-Cycloalkyl steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine ganz besonders bevorzugte Ausführungsform bezieht sich auf eine erfindungsgemäße Verbindung der Formel (I), worin Q für jeweils gegebenenfalls einfach mit Cyano substituiertes C₁-C₄-Alkyl oder C₃-C₆-Cycloalkyl, ganz besonders bevorzugt für gegebenenfalls einfach mit Cyano substituiertes C₃-C₆-Cycloalkyl wie z. B. C₃-Cycloalkyl steht, und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine bevorzugte Ausführungsform bezieht sich auf eine erfindungsgemäße Verbindung der Formel (I), worin R¹ für Wasserstoff oder C₁-C₄-Alkyl steht W für Sauerstoff steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine weitere bevorzugte Ausführungsform bezieht sich auf eine erfindungsgemäße Verbindung der Formel (I), worin R¹ für Wasserstoff oder C₁-C₄-Alkyl steht, W für Sauerstoff steht, Z¹ und Z² jeweils unabhängig voneinander für C₁-C₄-Halogenalkyl stehen und Z³ für C₁-C₄-Alkyl steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine weitere bevorzugte Ausführungsform bezieht sich auf eine erfindungsgemäße Verbindung der Formel (I), worin R¹ für Wasserstoff steht, R⁵ für Wasserstoff steht, W für Sauerstoff steht, Z¹ und Z² jeweils unabhängig voneinander für C₁-C₄-Halogenalkyl stehen und Z³ für C₁-C₄-Alkyl und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0075] (ohne diesen Paragraphen) definiert sind.

Eine weitere bevorzugte Ausführungsform bezieht sich auf eine erfindungsgemäße Verbindung der Formel (I), worin R¹ für Wasserstoff steht, R⁵ für Wasserstoff steht, W für Sauerstoff steht, Z¹ und Z² jeweils unabhängig voneinander für C₁-C₄-Halogenalkyl stehen und Z³ für C₁-C₄-Alkyl steht Q für jeweils gegebenenfalls einfach mit Cyano substituiertes oder jeweils gegebenenfalls einfach, zweifach, dreifach vierfach oder fünffach unabhängig voneinander mit Fluor, Chlor, Brom, Iod, substituiertes oder perfluoriertes oder perchloriertes C₁-C₄-Alkyl oder C₃-C₆-Cycloalkyl steht und ansonsten die übrigen Parameter einer erfindungsgemäßen Verbindung der Formel (I) wie in Paragraph [0006] definiert sind oder die übrigen Parameter einer erfindungsgemäßen Verbindung der Formel (I) wie in Paragraphen [0062] bis [0074] definiert sind.

Weiterhin bevorzugt sind Verbindungen der Formel (I) worin
- R¹: für Wasserstoff, oder jeweils gegebenenfalls ein- oder mehrfach unabhängig voneinander mit Halogen, Cyano, Alkoxy und Alkoxycarbonyl substituiertes C₁-C₆-Alkyl, C₂-C₆-Alkenyl, C₂-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, C₁-C₆-Alkoxy-C₁-C₆-alkyl, Aryl(C₁-C₃)-alkyl, Heteroaryl(C₁-C₃)-alkyl, steht;
die chemischen Gruppierungen
- A₁: für CR² oder Stickstoff,
- A₂: für Stickstoff,
- A₃: für CR³ oder Stickstoff, und
- A₄: für CR⁴ oder Stickstoff stehen,
wobei aber nicht mehr als drei der chemischen Gruppierungen A₁ bis A₄ gleichzeitig für Stickstoff stehen;
- R², R³ und R⁴: unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N*-*Di-*(C₁-C₆-alkyl)amino, C₁-C₆-Alkylsulfonylamino, oder *N*-(C₁-C₆-Alkyl)-C₁-C₆-alkylsulfonyl-amino stehen;
- W: für Sauerstoff oder Schwefel steht;
- Q: für Wasserstoff, Hydroxy, Formyl oder eine der jeweils gegebenenfalls unabhängig voneinander ein- oder mehrfach mit Hydroxy, Nitro, Amino, Halogen, Alkoxy, Cyano, Hydroxycarbonyl. Alkoxycarbonyl, Alkylcarbamoyl, Cycloalkylcarbamoyl oder Phenyl substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl, *N*-(C₁-C₆-Alkyl)amino, *N,N*-Di-(C₁-C₆-Alkyl)amino, oder *N*-(C₁-C₆-Alkylcarbonyl)amino steht; oder
- Q: für ein mit 0 - 4 Substituenten V substituierten Aryl oder für ein mit 0 - 4 Substituenten V substituierten 5 bzw. 6 gliedrigen Heteroaromaten steht, wobei
- V: unabhängig voneinander für Halogen, Cyano, Nitro, oder eine der jeweils gegebenenfalls unabhängig voneinander ein- oder mehrfach mit Hydroxy, Nitro, Amino, Halogen, Alkoxy, Cyano oder Phenyl substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆- Alkylsulfonyl, *N*-(C₁-C₆-Alkyl)amino oder *N,N*-Di(C₁-C₆-Alkyl)amino steht;
- R⁵: unabhängig voneinander für Wasserstoff, Halogen, Cyano, Nitro, Amino oder jeweils gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆-Alkyl)amino oder *N,N-*Di-(C₁-C₆-Alkyl)amino steht;
- Z¹: für ein jeweils gegebenenfalls mit Halogen substituiertes C₁-C₆-Alkyl steht;
- Z²: für Wasserstoff, Halogen, Cyano, Nitro, Amino oder ein jeweils gegebenenfalls mit Halogen substituiertes C₁-C₆-Alkyl, C₁-C₆-Alkylcarbonyl, C₁-C₆-Alkylsulfanyl, C₁-C₆-Alkylsulfinyl oder C₁-C₆-Alkylsulfonyl steht; und
- Z³: für Wasserstoff oder ein jeweils gegebenenfalls mit Halogen substituiertes C₁-C₆-Alkyl, C₃-C₆-Cycloalkyl, C₂-C₄-Alkenyl, C₂-C₄-Alkinyl, Aryl oder Hetaryl steht.

Besonders bevorzugt sind Verbindungen der Formel (I) worin
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CR² oder Stickstoff,
- A₂: für Stickstoff,
- A₃: für CR³ oder Stickstoff, und
- A₄: für CR⁴ oder Stickstoff stehen,
wobei aber nicht mehr als drei der chemischen Gruppierungen A₁ bis A₄ gleichzeitig für Stickstoff stehen;
- R² und R⁴: unabhängig voneinander für Wasserstoff, Methyl, Fluor und Chlor stehen; und
- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
- W: für Sauerstoff oder Schwefel steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N-*Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
- R⁵: unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
- Z¹: für Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl steht;
- Z²: für Wasserstoff, Halogen, Cyano, Nitro,, Amino, Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl steht; und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl steht.

Insbesondere bevorzugt sind Verbindungen der allgemeinen Formel (I) worin
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CH,
- A₂: für Stickstoff,
- A₃: für CR³, und
- A₄: für CH stehen;

- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
- W: für Sauerstoff steht; und
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, l-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N-*Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
- R⁵: für Wasserstoff, Methyl, Ethyl, 2-Methylethyl, tert.-Butyl, Fluor, Chlor, Brom, Iod, Nitro, Trifluormethyl, Amino steht;
- Z¹: für Trifluormethyl oder Pentafluorethyl steht;
- Z²: für Trifluormethyl, Difluormethyl, Nitro, Methylsulfanyl, Methylsulfinyl, Methylsulfonyl, Fluor, Chlor, Brom, Cyano oder Iod steht; und
- Z³: für Methyl, Ethyl, n-Propyl oder Wasserstoff steht.

Insbesondere bevorzugt sind ferner die Verbindungen, die jeweils durch Formeln (Ia) dargestellt werden können: 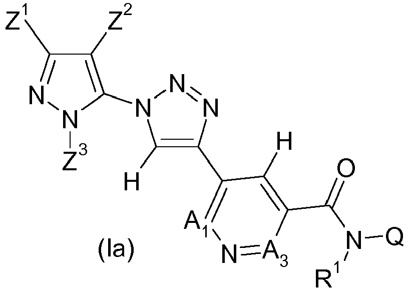 worin
- R¹: für Wasserstoff, Methyl oder Ethyl, bevorzugt Wasserstoff, steht; und
- A₁: für C-H oder C-(C₁-C₄-Alkyl) steht; bevorzugt für C-H steht; und
- A₃: für CR³ steht; worin
- R³: für Chlor, Fluor, Brom, Iod, C₁-C₄-Alkoxy oder Wasserstoff steht; bevorzugt für Chlor, Wasserstoff oder C₁-C₄-Alkoxy steht; mehr bevorzugt für Chlor, Wasserstoff oder Methoxy steht; und
- Z¹: für C₁-C₄-Alkyl oder C₁-C₄-Halogenalkyl steht; bevorzugt für Trifluormethyl oder Pentafluorethyl steht; ganz besonders bevorzugt für Pentafluoroethyl steht; und
- Z²: für C₁-C₄-Halogenalkyl, Nitro, C₁-C₄-Alkylsulfanyl, C₁-C₄-Alkylsulfinyl, C₁-C₄-Alkylsulfonyl, Fluor, Chlor, Brom, Cyano oder Iod steht, bevorzugt für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
- Z³: für Methyl, Ethyl, n-Propyl oder Wasserstoff steht; bevorzugt für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Weiterhin insbesondere bevorzugt sind ferner die Verbindungen der Formel (Ib) 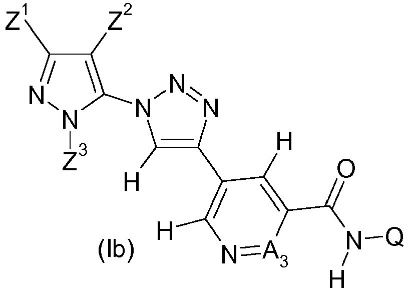 worin
- A₃: für CR³ steht; worin
- R³: für Chlor, Wasserstoff oder C₁-C₄-Alkoxy steht; mehr bevorzugt für Chlor, Wasserstoff oder Methoxy steht; und
- Z¹: für Trifluormethyl oder Pentafluorethyl steht; ganz besonders bevorzugt für Pentafluoroethyl steht; und
- Z²: für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl, C₁-C₄-Alkoxy C₁-C₄-Halogenalkoxy substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Weiterhin insbesondere bevorzugt sind ferner die Verbindungen der Formel (Ib), worin
- A₃: für CR³ steht; worin
- R³: für Chlor, Wasserstoff oder C₁-C₄-Alkoxy steht; mehr bevorzugt für Chlor, Wasserstoff oder Methoxy steht; und
- Z¹: für Pentafluoroethyl steht; und
- Z²: für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

Weiterhin insbesondere bevorzugt sind ferner die Verbindungen der Formel (Ib) worin
- A₃: für CR³ steht; worin
- R³: für Chlor, Wasserstoff oder C₁-C₄-Alkoxy steht; mehr bevorzugt für Chlor, Wasserstoff oder Methoxy steht; und
- Z¹: für Pentafluoroethyl steht; und
- Z²: für Trifluomethyl steht; und
- Z³: für Methyl steht; und
- Q: für Wasserstoff oder jeweils gegebenenfalls einfach, zweifach, dreifach oder vierfach unabhängig voneinander mit Cyano, Fluor, Chlor, Brom, Iod, substituiertes C₁-C₄-Alkyl, C₁-C₄-Halogenalkyl, C₃-C₆-Cycloalkyl, Phenyl, Benzyl, Pyridin, Pyrazol, Thiazol steht.

Weiterhin insbesondere bevorzugt sind ferner die Verbindungen der Formel (Ib) worin
- A₃: für C-R³ steht; und
- R³: für Chlor, Wasserstoff oder C₁-C₄-Alkoxy steht; mehr bevorzugt für Chlor, Wasserstoff oder Methoxy steht; und
- Z¹: für Pentafluoroethyl steht; und
- Z²: für Trifluomethyl steht; und
- Z³: für Methyl steht; und
- Q: für jeweils gegebenenfalls einfach mit Cyano substituiertes oder jeweils gegebenenfalls einfach, zweifach, dreifach vierfach oder fünffach unabhängig voneinander mit Fluor, Chlor, Brom, Iod, substituiertes C₁-C₄-Alkyl oder C₃-C₆-Cycloalkyl steht.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Beschrieben werden auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Comitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Be-tracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite, Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N- {2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl] -1H-imidazol-1 -carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazo1-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1) Andoprim, (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb,, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3 -methoxy-4-(prop-2-yn-1 -yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl} -2-phenylacetamid, (15.85) N-{(Z)-[(Cyclopropylmethoxy)imino][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1 -yl)-1,3 -thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1 -methyl-N- [4'-(trifluormethyl)biphenyl-2-yl] -1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N- [4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N- [4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N- [(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'- {5-Brom-6-[(cis-4-isopropylcyclohexyl)oxy]-2-methylpyridin-3-yl}-N-ethyl-N-methylimidoformamid, (15.156) N'- {5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus*, insbesondere *B. cereus* Stamm CNCM I-1562 oder *Bacillus firmus*, Stamm I-1582 (Accession number CNCM I-1582) oder *Bacillus pumilus*, insbesondere Stamm GB34 (Accession No. ATCC 700814) und Stamm QST2808 (Accession No. NRRL B-30087), oder *Bacillus subtilis*, insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis*, insbesondere *B. thuringiensis* subspecies *israelensis* (serotype H-14), Stamm AM65-52 (Accession No. ATCC 1276), oder *B. thuringiensis subsp. aizawai*, insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana*, insbesondere Stamm ATCC 74040, *Coniothyrium minitans,* insbesondere Stamm CON/M/91-8 (Accession No. DSM-9660), *Lecanicillium spp*., insbesondere Stamm HRO LEC 12, *Lecanicillium lecanii*, (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus (*neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp*., *Burkholderia spp*., insbesondere *Burkholderia cepacia* (ehemals bekannt als *Pseudomonas cepacia*), *Gigaspora spp*., oder *Gigaspora monosporum*, *Glomus spp*., *Laccaria spp*., *Lactobacillus buchneri*, *Paraglomus spp*., *Pisolithus tinctorus*, *Pseudomonas spp*., *Rhizobium spp*., insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanzen, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Stemostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenzbrechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsmethoden

Die erfindungsgemäßen Verbindungen können nach üblichen, dem Fachmann bekannten Methoden hergestellt werden.

Im Reaktionsschema 1 sind die allgemeinen Darstellungsverfahren für die erfindungsgemäßen Verbindungen (**I**) und (**I-1**) abgebildet.

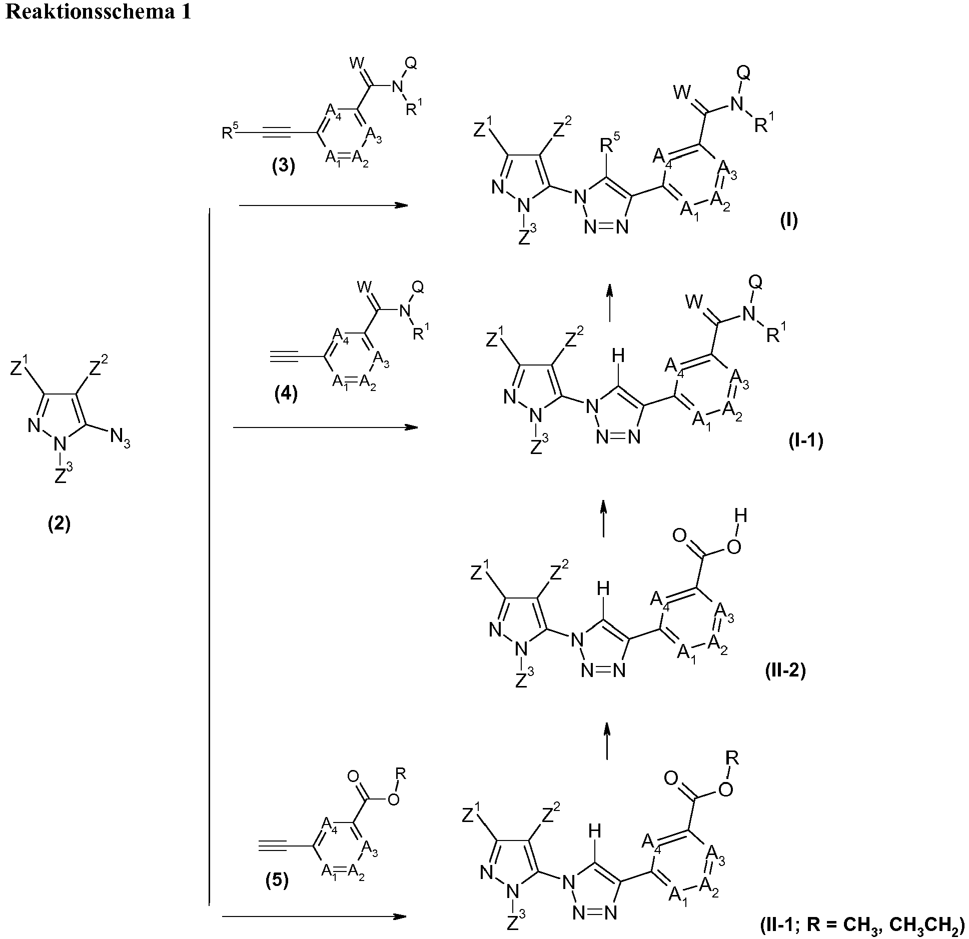

Die Reste A₁-A₄, Q, W, R¹, R⁵ und Z¹-Z³ haben die oben beschriebenen Bedeutungen.

Erfindungsgemäße Verbindungen der allgemeinen Struktur **(I)** oder **(I-1)** können nach dem aus WO2012107434-A1 bekannten Verfahren aus einem Pyrazolylazid **(2)** und bestimmten Acetylen-Verbindungen **(3)** oder **(4)** im Zuge einer [2+3]-Cycloaddition hergestellt werden. Alternativ können mit den entsprechenden Carbonsäureestern **(5)** die Intermediate **(II-1)** hergestellt werden, aus denen die Verbindungen **(II-2)**, **(I-1)** oder **(I)** nach dem Fachmann bekannten Verfahren sukzessiv gewonnen werden können.

Insbesondere bevorzugt sind Intermediate der allgemeinen Formel (II) definiert, 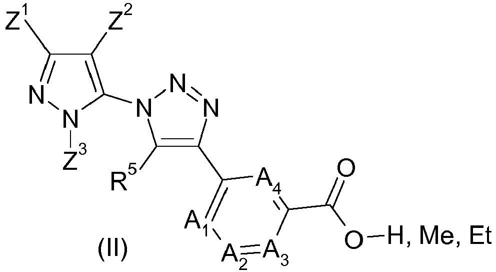 in denen O-Me für -O-CH₃ steht und -O-Et für -O-CH₂-CH₃ steht,
die chemischen Gruppierungen
- A₁: für CH,
- A₂: für Stickstoff,
- A₃: für CR³, und
- A₄: für CH stehen;

- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N*,*N*-Dimethylamino, *N*-Ethylamino, *N*,*N*-Diethylamino, Methylsulfonylamino, *N*-Methyl-methylsulfonylamino steht;
- R⁵: für Wasserstoff, Methyl, Ethyl, 2-Methylethyl, tert.-Butyl, Trifluormethyl steht;
- Z¹: für Trifluormethyl oder Pentafluorethyl steht;
- Z²: für Trifluormethyl, Difluormethyl, Nitro, Methylsulfanyl, Methylsulfinyl, Methylsulfonyl, Fluor, Chlor, Brom, Cyano oder Iod steht; und
- Z³: für Methyl, Ethyl, n-Propyl oder Wasserstoff steht.

In einer bevorzugten Ausführungsform haben die Parameter in einem Intermediate der allgemeinen Formel (II) die folgenden Bedeutungen:
die chemischen Gruppierungen
- A₁: für CH,
- A₂: für Stickstoff,
- A₃: für CR³, und
- A₄: für CH stehen;

- R³: für Wasserstoff, Fluor, Chlor, Brom, Iod, Methyl, Ethyl, Methoxy, Ethoxy, n-Propoxy, 1-Methylethoxy steht;
- R⁵: für Wasserstoff steht;
- Z¹: für Trifluormethyl oder Pentafluorethyl steht;
- Z²: für Trifluormethyl, Difluormethyl steht; und
- Z³: für Methyl, Ethyl, oder n-Propyl steht.

Insbesondere bevorzugt sind Intermediate der allgemeinen Formel (III) definiert, 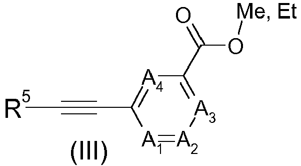 in denen O-Me für -O-CH₃ steht und -O-Et für -O-CH₂-CH₃ steht,
die chemischen Gruppierungen
- A₁: für CH,
- A₂: für Stickstoff,
- A₃: für CR³, und
- A₄: für CH stehen;

- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N*,*N*-Dimethylamino, *N*-Ethylamino, *N*,*N*-Diethylamino, Methylsulfonylamino, *N*-Methyl-methylsulfonylamino steht; und
- R⁵: für Wasserstoff, Methyl, Ethyl, 2-Methyl, Ethyl, 2-Methylethyl, tert.-Butyl, Trifluormethyl steht.

In einer bevorzugten Ausführungsform haben die Parameter in einem Intermediate der allgemeinen Formel (III) die folgenden Bedeutungen:
die chemischen Gruppierungen
- A₁: für CH,
- A₂: für Stickstoff,
- A₃: für CR³, und
- A₄: für CH stehen;

- R₃: für Wasserstoff, Fluor, Chlor, Brom, Iod, Methyl, Ethyl, Methoxy, Ethoxy, n-Propoxy, l-Methylethoxy steht;
- R₅: für Wassertoff steht.

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agens in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag GmbH & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der t-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-t-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N*,*N*-Dimethyl-formamid, *N*,*N*-Dipropyl-formamid, *N*,*N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N*,*N'*-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N,N-*Dimethylanilin, *N,N*-Dimethyl-toluidin, *N*,*N*-Dimethyl-p-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N*,N',N'-Tetramethylendiamin, *N,N*,N',N'-Tetraethylendiamin, Chinoxalin, N-Propyl-diisopropylamin, N-Ethyl-diisopropylamin, *N,N*-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

### Weiterhin eignen sich auch Schutzgruppen

vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), t-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), t-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxybenzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), t-Butyldimethylsilylether (TBDMS-OR), t-Butyldiphenylsilylether(TBDPS-OR), Tribenzylsilylether, Tri-para-xylylsilylether, Triphenylsilylether (TPS-OR), Diphenylmethylsilylether (DPMS-OR), Di-t-butylmethylsilylether (DTBMS-OR), Tris(trimethylsilyl)silylether (Sisylether), Di-t-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), t-Butylmethoxyphenyl-silylether (TBMPS-OR), t-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxopentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMS-OR), 2-(Phenylsulfonyl)-ethylcarbonat (Ps-OR), 2-(Triphenylphosphonio)-ethylcarbonat (Peoc-OR), t-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und
vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , NickelKatalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer N-Oxide und t-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. t-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Herstellungsbeispiele

Mit Hilfe der oben beschriebenen Darstellungsverfahren A bis C wurden die in den Tabellen 1 - 3 aufgeführten Verbindungen dargestellt.

Die NMR Daten wurden mit einem AV-III 300 MHz (Firma Bruker) aufgenommen. Die MS-Daten wurden mit einem LC-MS2020 (Firma Shimadzu) aufgenommen (Wasser/Acetonitril-Gradient, 0,05% Trifluoressigsäure, Chromatographiesäule: Shimadzu shim-pack XR-ODS, Länge 50 mm, Durchmesser 3 mm, Partikelgröße 2.2 µm, Ofentemperatur 40 °C, Massenspektroskopie nach ESI-Verfahren, 250 °C, Detektor-Spannung +1.05 kV, Angabe der Daten für *m*/*z*: (ESI⁺) [M+H]).

### Stufe 1

### 5-Azido-1-methyl-3-(pentafluorethyl)-4-(trifluormethyl)-1H-pyrazol

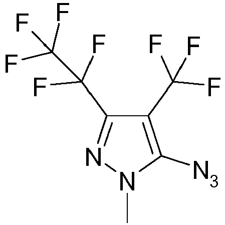

1 g (3.5 mmol) 5-Fluor-1-methyl-3-(pentafluoroethyl)-4-(trifluormethyl)-1H-pyrazol wird in 50 mL DMSO gelöst und mit 250 mg (3.85 mol) Natriumazid versetzt. Das Reaktionsgemisch wird bei Raumtemperatur über Nacht gerührt. Anschließend wird mit 50 mL Wasser verdünnt und dreimal mit Essigsäureethylester extrahiert. Die vereinigten organischen Extrakte werden dreimal mit gesättigter wässriger NaCl-Lösung gewaschen und dann über Natriumsulfat getrocknet, anschließend filtriert. Das Filtrat wird im Vakuum eingeengt. Das Produkt wird ohne weitere Aufreinigung weiter umgesetzt. Ausbeute: 0.5 g (46 % d. Th.) als bräunliches Öl.
¹H NMR (300 MHz, CDCl₃, 25°C): 3.79 (3H, s).

### Stufe 2

### 2-Chlor-5-ethinyl-pyridin-3-carbonsäuremethylester

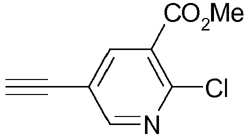

20 g (67.23 mmol) 2-Chlor-5-iod-pyridin-3-carbonsäuremethylester werden unter Inertgasatmosphäre zusammen mit 200 mL Triethylamin, 8 g (81.45 mmol) Trimethylsilylacetylen, 3 g (15.71 mmol) CuI, 4.3 g (16.41 mmol) Triphenylphosphin und 3.04 g (4.33 mol) Pd(PPh₃)₂Cl₂ für 12 Stunden bei 50 °C gerührt. Anschließend wird das Reaktionsgemisch mit 40 mL Methanol und 4 g (33.61 mmol) Kaliumcarbonat weitere 2 Stunden bei Raumtemperatur gerührt. Die Feststoffe werden abfiltriert, das Filtrat wird mit Wasser verdünnt und und dreimal mit Essigsäureethylester extrahiert. Die vereinigten organischen Extrakte werden dreimal mit gesättigter wässriger NaCl-Lösung gewaschen und dann über Natriumsulfat getrocknet, anschließend filtriert. Das Filtrat wird im Vakuum eingeengt. Der Rückstand wird über Kieselgel mit einem Essigsäureethylester/Cyclohexan-Gradienten chromatographiert. Ausbeute: 7.5 g (57% d. Th.) als brauner Feststoff.
¹H NMR (300 MHz, CDCl₃, 25°C): 8.70-8.71 (1H, d), 8.33-8.34 (1H, d), 4.68 (1H, s), 3.89 (3H, s).

### Stufe 3

### 2-Chlor-5-[1-[2-methyl-5-(1,1,2,2,2-pentafluorethyl)-4-(trifluormethyl)pyrazol-3-yl]triazol-4-yl]pyridin-3 -carbonsäuremethylester

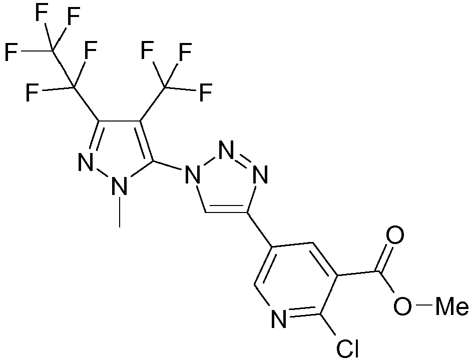

1.57 g (5.08 mmol) 5-Azido-1-methyl-3-(pentafluorethyl)-4-(trifluormethyl)-1H-pyrazol (Stufe 1) und 0.82 g (4.23 mmol) 2-Chlor-5-ethinyl-pyridin-3-carbonsäuremethylester (Stufe 2) werden unter Inertgasatmospäre in 30 mL Acetonitril gelöst und mit 0.65 g (4.23 mmol) Diisopropylethylamin und 0.1 g (0.42 mmol) CuI versetzt. Das Reaktionsgemisch wird 12 Stunden bei Raumtemperatur gerührt, anschließend wird mit 50 mL Wasser verdünnt und dreimal mit Essigsäureethylester extrahiert. Die vereinigten organischen Extrakte werden dreimal mit gesättigter wässriger NaCl-Lösung gewaschen und dann über Natriumsulfat getrocknet, anschließend filtriert. Das Filtrat wird im Vakuum eingeengt. Das Produkt wird ohne weitere Aufreinigung weiter umgesetzt.
*m*/*z*: (ESI⁺) [M+H]⁺ = 505.

### Stufe 4

### 2-Chlor-5-[1-[2-methyl-5-(1,1,2,2,2-pentafluorethyl)-4-(trifluormethyl)pyrazol-3-yl]triazol-4-yl]pyridin-3-carbonsäure

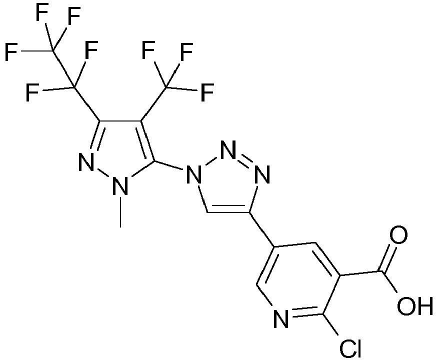

1.02 g (2.2 mmol) 2-Chlor-5-[1-[2-methyl-5-(1,1,2,2,2-pentafluorethyl)-4-(trifluormethyl)-pyrazol-3-yl]triazol-4-yl]pyridin-3-carbonsäuremethylester (Stufe 3) werden in 15 mL THF mit 300 mg (7.5 mmol) NaOH und 5 mL Wasser versetzt. Das Reaktionsgemisch wird 12 Stunden bei Raumtemperatur gerührt. Anschließend wird das Reaktionsgemisch im Vakuum eingeengt und mit 3M HCl auf pH 3-4 gebracht. Der Niederschlag wird abgesaugt und an der Luft getrocknet und ohne weitere Aufreinigung weiter umgesetzt.
*m*/*z*: (ESI⁺) [M+H]⁺ = 491; 532 (+ACN).

### Stufe 5

### 2-Chlor-N-cyclopropyl-5-[1-[2-methyl-5-(1,1,2,2,2-pentafluorethyl)-4-(trifluormethyl)pyrazol-3-yl]triazol-4-yl]pyridin-3-carboxamid

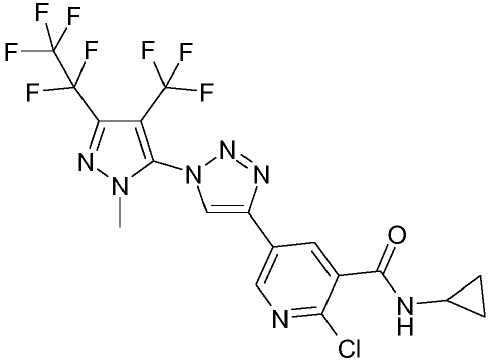

490 mg (1 mmol) 2-Chlor-5-[1-[2-methyl-5-(1,1,2,2,2-pentafluorethyl)-4-(trifluormethyl) pyrazol-3-yl]triazol-4-yl]pyridin-3-carbonsäure (Stufe 4) werden in 20 mL Dioxan gelöst und mit 119 mg (2.1 mmol) Cyclopropylamin, 390 mg Diisopropylethylamin und 321 mg (1.01 mmol) T3P (2,4,6-Tripropyl-1,3,5,2,4,6-trioxatriphosphorinan-2,4,6-trioxid) versetzt. Das Reaktionsgemisch wird 12 h bei 50 °C gerührt und anschließend im Vakuum eingeengt. Dannach wird mit Wasser verdünnt und dreimal mit Essigsäureethylester extrahiert. Die vereinigten organischen Extrakte werden dreimal mit gesättigter wässriger NaCl-Lösung gewaschen und dann über Natriumsulfat getrocknet, anschließend filtriert. Das Filtrat wird im Vakuum eingeengt und mittels Flash-Chromatographie aufgereinigt. Ausbeute: 145.5 mg (27.5 % d. Th.) als farbloser Feststoff.
¹H NMR (300 MHz, d6-DMSO, 25°C): 9.35 (s, 1H), 9.06 (s, 1H), 9.77 (d, NH), 8.42 (s, 1H), 3.87 (s, 3H), 2.90 - 2.82 (m, 1H), 0.78 - 0.75 (m, 2H), 0.74 - 0.71 (m, 2H).
*m*/*z*: (ESI⁺) [M+H]⁺ = 530; 571 (+ACN).

**Tabelle 1**

| | | | | | | | | | | |
|---|---|---|---|---|---|---|---|---|---|---|
| 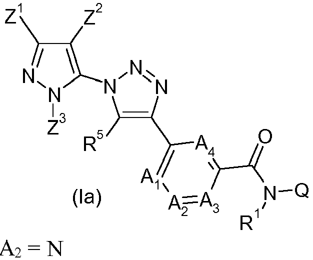 | | | | | | | | | | |

| **Bsp.-Nr.** | **Z³** | **Z¹** | **Z²** | **R¹** | **R⁵** | **A₁** | **A₃** | **A₄** | **Q** | **NMR Daten (¹H, NMR, 300 MHz, d6-DMSO, δ ppm)** |
|---|---|---|---|---|---|---|---|---|---|---|
| Ia-1 | CH₃ | CF₂CF₃ | CF₃ | H | H | C-H | C-Cl | C-H | 1-Cyano-cyclopropyl | 9.67 (s, 1H), 9.36 (s, 1H), 9.11 (s, 1H), 8.52 (s, 1H), 3.87 (s, 3H), 1.65 - 1.60 (t, 2H), 1.34 - 1.29 (t, 2H). |
| Ia-2 | CH₃ | CF₂CF₃ | CF₃ | H | H | C-H | C-Cl | C-H | Cyclopropyl | 9.35 (s, 1H), 9.06 (s, 1H), 9.77 (d, NH), 8.42 (s, 1H), 3.87 (s, 3H), 2.90 - 2.82 (m, 1H), 0.78 - 0.75 (m, 2H), 0.74 - 0.71 (m, 2H). |
| Ia-3 | CH₃ | CF₂CF₃ | CF₃ | H | H | C-H | C-CH₃ | C-H | 1-Cyano-cyclopropyl | 9.51 (s, 1H), 9.31 (s, 1H), 9.13 (s, 1H), 8.30 (s, 1H), 3.87 (s, 3 H), 2.58 (s, 3H), 1.62 - 1.58 (t, 2H), 1.35 - 1.30 (t, 2H). |
| Ia-4 | CH₃ | CF₂CF₃ | CF₃ | H | H | C-H | C-CH₃ | C-H | Cyclopropyl | 9.30 (s, 1H), 9.08 (s, 1H), 8.63 (d, NH), 8.20 (s, 1H), 3.87 (s, 3H), 2.92 - 2.83 (m, 1H), 2.56 (s, 3H), 0.76 - 0.70 (m, 2H), 0.59 - 0.55 (m, 2H). |
| Ia-5 | CH₃ | CF₂CF₃ | CF₃ | H | H | C-H | C-OCH₃ | C-H | 1-Cyano-cyclopropyl | 9.26 (s, 1H), 9.15 (s, 1H), 8.90 (s, 1H), 8.59 (s, 1H), 4.03 (s, 3 H), 3.87 (s, 3H), 1.62 - 1.58 (t, 2H), 1.34 - 1.29 (t, 2H). |
| Ia-6 | CH₃ | CF₂CF₃ | CF₃ | H | H | C-H | C-OCH₃ | C-H | Cyclopropyl | 9.24 (s, 1H), 8.86 (s, 1H), 8.50 (s, 1H), 8.32 (d, NH), 4.00 (s, 3H), 3.87 (s, 3H), 2.90 - 2.84 (m, 1H), 0.76 - 0.72 (m, 2H), 0.60 - 0.57 (m, 2H). |

**Tabelle 2: Intermediate**

| | | | | | | | | | | |
|---|---|---|---|---|---|---|---|---|---|---|
| 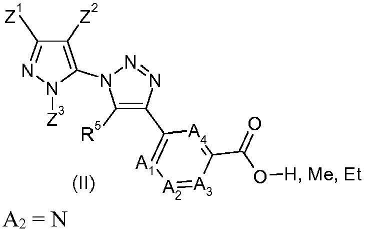 | | | | | | | | | | |

| **Bsp.-Nr.** | **Z³** | **Z¹** | **Z²** | **R¹** | **R⁵** | **A₁** | **A₃** | **A₄** | **OR** | **NMR-Daten (¹H, NMR, 300 MHz, d6-DMSO, δ ppm) oder *m*/*z*: (ESI⁺) [M+H]** |
|---|---|---|---|---|---|---|---|---|---|---|
| II-1 | CH₃ | CF₂CF₃ | CF₃ | H | H | C-H | C-Cl | C-H | OMe | *m*/*z*: (ESI⁺) [M+H]⁺ = 505 |
| II-2 | CH₃ | CF₂CF₃ | CF₃ | H | H | C-H | C-Cl | C-H | OH | *m*/*z*: (ESI⁺) [M+H] ⁺ = 491; 532 (+ACN) |
| II-3 | CH₃ | CF₂CF₃ | CF₃ | H | H | C-H | C-CH₃ | C-H | OEt | *m*/*z*: (ESI⁺) [M+H]⁺ = 499 |
| II-4 | CH₃ | CF₂CF₃ | CF₃ | H | H | C-H | C-CH₃ | C-H | OH | *m*/*z*: (ESI⁺) [M+H] ⁺ = 471; 512 (+ACN) |
| II-5 | CH₃ | CF₂CF₃ | CF₃ | H | H | C-H | C-OCH₃ | C-H | OMe | |
| II-6 | CH₃ | CF₂CF₃ | CF₃ | H | H | C-H | C-OCH₃ | C-H | OH | |

**Tabelle 3: Intermediate**

| | | | | | | |
|---|---|---|---|---|---|---|
| 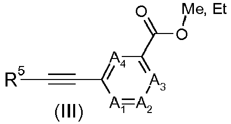 | | | | | | |
| A₂ = N | | | | | | |

| **Bsp.-Nr.** | **R⁵** | **A₁** | **A₃** | **A₄** | **OR** | **NMR-Daten (¹H, NMR, 300 MHz, CDCl₃, δ ppm)** |
|---|---|---|---|---|---|---|
| III-1 | H | C-H | C-Cl | C-H | OMe | 8.70-8.71 (1H, d), 8.33-8.34 (1H, d), 4.68 (1H, s), 3.89 (3H, s). |
| III-2 | H | C-H | C-CH₃ | C-H | OEt | |
| III-3 | H | C-H | C-OCH₃ | C-H | OMe | |

### Biologische Beispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 500 g/ha mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 500 g/ha: Ia-3, Ia-4

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 500 g/ha mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 100 g/ha: Ia-1, Ia-2, Ia-3, Ia-4, Ia-5

Bei diesem Test zeigen z.B. die folgenden Verbindungen der Herstellungsbeispiele eine Wirkung von 80% bei einer Aufwandmenge von 20 g/ha: Ia-6

### Boophilus microplus - Tauchtest

| | |
|---|---|
| Testtiere: | Rinderzecken (*Boophilus microplus*) Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: Ia-1, Ia-2, Ia-4

### Boophilus microplus-Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: Ia-1, Ia-2, Ia-3, Ia-4, Ia-5, Ia-6

### Boophilus microplus - Diptest

| | |
|---|---|
| Testtiere: | Rinderzecken (*Boophilus microplus*) Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: Ia-1, Ia-2, Ia-4

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 100g/ha: Ia-3

Bei diesem Test zeigen z. B. die folgenden Verbindungen der Herstellungsbeispiele Wirkung von 90% bei einer Aufwandmenge von 100g/ha: Ia-2, Ia-4

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100g/ha: Ia-1, Ia-2, Ia-3, Ia-4, Ia-5, Ia-6

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100g/ha: Ia-2, Ia-4, Ia-5

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 100g/ha: Ia-2, Ia-3, Ia-4

Bei diesem Test zeigen z. B. die folgenden Verbindungen der Herstellungsbeispiele eine Wirkung von 90% bei einer Aufwandmenge von 100g/ha: Ia-1

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100 ppm: Ia-1, Ia-2, Ia-3, Ia-4, Ia-5, Ia-6

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100 % bei einer Aufwandmenge von 100 ppm: Ia-2, Ia-4, Ia-5, Ia-6

Bei diesem Test zeigen z.B. die folgenden Verbindungen der Herstellungsbeispiele eine Wirkung von 80 % bei einer Aufwandmenge von 100 ppm: Ia-3

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100 % bei einer Aufwandmenge von 100 ppm: Ia-2, Ia-4, Ia-5, Ia-6

Bei diesem Test zeigen z.B. die folgenden Verbindungen der Herstellungsbeispiele eine Wirkung von 95 % bei einer Aufwandmenge von 100 ppm: Ia-3

### Meloidogyne incognita-Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20 ppm: Ia-4

### Amblvomma hebaraeum -Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100 % bei einer Aufwandmenge von 100 ppm: Ia-1, Ia-2, Ia-4

## Patentansprüche

1. Verbindung der Formel (I) 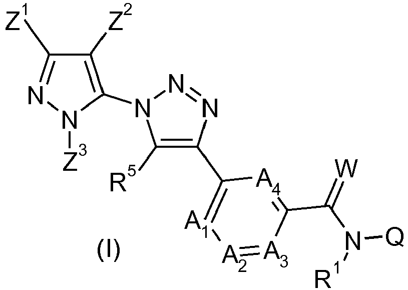 worin
R¹ für Wasserstoff, jeweils gegebenenfalls substituiertes C₁-C₆-Alkyl, C₂-C₆-Alkenyl, C₂-C₆-Alkinyl, C₃-C₇-Cycloalkyl, C₃-C₇-Cycloalkyl-C₁-C₆-alkyl C₁-C₆-Alkylcarbonyl, C₁-C₆-Alkoxycarbonyl, C₁-C₆-Alkoxy-C₁-C₆-alkyl, Aryl-C₁-C₆-alkyl, Heteroaryl-C₁-C₆-alkyl steht;
die chemischen Gruppierungen
A₁ für CR² oder Stickstoff,
A₂ für Stickstoff,
A₃ für CR³ oder Stickstoff und
A₄ für CR⁴ oder Stickstoff stehen,
wobei aber nicht mehr als drei der chemischen Gruppierungen A₁ bis A₄ gleichzeitig für Stickstoff stehen;
R², R³ und R⁴ unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N*-Di-(C₁-C₆-Alkyl)amino, C₁-C₆-Alkylsulfonyl-amino, oder *N*-(C₁-C₆-Alkyl)-C₁-C₆-alkylsulfonyl-amino stehen;
W für Sauerstoff oder Schwefel steht;
Q für Wasserstoff, Formyl, Hydroxy, Amino oder eine der jeweils gegebenenfalls substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
Q für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
V für Halogen, Cyano, Nitro, oder eine der jeweils gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-Alkyl, C₁-C₆-Alkylsulfanyl, C₁-C₆-Alkylsulfinyl, C₁-C₆-Alkylsulfonyl, *N*-(C₁-C₆-Alkyl)amino, oder *N,N*-Di(C₁-C₆-Alkyl)amino steht;
R⁵ unabhängig voneinander für Wasserstoff, Halogen, Cyano, Nitro, Amino, jeweils gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆)-Alkylamino oder *N,N*-Di-(C₁-C₆-Alkyl)amino steht;
Z¹ für ein gegebenenfalls substituiertes C₁-C₆-Alkyl steht;
Z² für Wasserstoff, Halogen, Cyano, Nitro oder jeweils gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl steht; und
Z³ für Wasserstoff oder jeweils gegebenenfalls substituiertes C₁-C₆-Alkyl, C₃-C₇-Cycloalkyl, C₃-C₆-Alkenyl, C₃-C₆-Alkinyl, Aryl oder Hetaryl steht,
wobei "gegebenenfalls substituiert", soweit keine spezifischen Substituenten angegeben sind, heißt, dass die entsprechende Gruppe einfach oder mehrfach mit einem Substituenten M¹ substituiert sein kann, wobei bei Mehrfachsubstitutionen die Substituenten M¹ gleich oder verschieden sein können und die Substituenten M¹ ausgewählt sind aus der Gruppe bestehend aus: Halogen, Hydroxy, Nitro, Formyl, Carboxy, Cyano, Amino, Isocyano, Azido, (C₁-C₄)-Alkyl, (C₁-C₄)-Halogenalkyl, (C₂-C₄)-Alkenyl, (C₂-C₄)-Alkinyl, (C₃-C₆)-Cycloalkyl, (C₁-C₄)-Alkoxy, (C₁-C₄)-Halogenalkoxy, (C₁-C₄)-Alkoxy-(C₁-C₄)-alkoxy, (C₁-C₄)-Alkoxy-(C₁-C₆)-alkyl, *N*-(C₁-C₄)-Alkoxy-imino-(C₁-C₃)-alkyl, (C₁-C₄)-Alkylsulfanyl, (C₁-C₄)-Halogenalkylsulfanyl, (C₁-C₄)-Alkoxycarbonyl, (C₁-C₄)-Alkylcarbonyl, Carbamoyl, C₁-C₄-Alkylcarbamoyl, C₃-C₇-Cycloalkylcarbamoyl, Mono- und *N,N*-Di(C₁-C₄)-alkylaminocarbonyl, Amino, (C₁-C₆)-Acylamino, Mono- und *N,N*-Di(C₁-C₄)-alkylamino, Tri(C₁-C₄)-alkylsilyl, (C₃-C₆)-Cycloalkyl, C₆-Aryl, Heterocyclyl mit 3 bis 6 Ringatomen, wobei jeder der letztgenannten cyclischen Gruppen auch über Heteroatome oder eine divalente funktionelle CH₂-, oder C₂H₄-Gruppe gebunden sein kann, (C₁-C₄)-Alkylsulfinyl, wobei beide Enantiomere der (C₁-C₄)-Alkylsulfinylgruppe umfasst sind, (C₁-C₄)-Alkylsulfonyl, (C₁-C₄)-Alkylphosphinyl, (C₁-C₄)-(C₁-C₄)-Alkylsulfanyl-(C₁-C₄)-alkyl, (C₁-C₄)-Alkoxy-(C₁-C₄)-alkyl, Mono- und *N,N*-Di(C₁-C₄)-alkyl-amino(C₁-C₄)-alkyl und Hydroxy(C₁-C₄)-alkyl, wobei die Reste M¹ unsubstituiert sein können oder gegebenenfalls, sofern sie kohlenwasserstoffhaltige oder stickstoffwasserstoffhaltige Anteile enthalten, mit einem oder mehreren, vorzugsweise 1, 2 oder 3 Resten M² substituiert sein können, wobei M² unabhängig voneinander ausgewählt ist aus der Gruppe bestehend aus Amino, Hydroxy, Halogen, Nitro, Cyano, Isocyano, Mercapto, Isothiocyanato, Carboxy und Carboamid.

2. Eine Verbindung der Formel (I) gemäß Anspruch 1 worin
R¹ für Wasserstoff, oder jeweils gegebenenfalls ein- oder mehrfach unabhängig voneinander mit Halogen, Cyano, Alkoxy und Alkoxycarbonyl substituiertes C₁-C₆-Alkyl, C₂-C₆-Alkenyl, C₂-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, C₁-C₆-Alkoxy-C₁-C₆-alkyl, Aryl(C₁-C₃)-alkyl, Heteroaryl(C₁-C₃)-alkyl, steht;
die chemischen Gruppierungen
A₁ für CR² oder Stickstoff,
A₂ für Stickstoff,
A₃ für CR³ oder Stickstoff, und
A₄ für CR⁴ oder Stickstoff stehen,
wobei aber nicht mehr als drei der chemischen Gruppierungen A₁ bis A₄ gleichzeitig für Stickstoff stehen;
R², R³ und R⁴ unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino, *N,N*-Di-(C₁-C₆-alkyl)amino, C₁-C₆-Alkylsulfonylamino, oder *N*-(C₁-C₆-Alkyl)-C₁-C₆-alkylsulfonyl-amino stehen;
W für Sauerstoff oder Schwefel steht;
Q für Wasserstoff, Hydroxy, Formyl oder eine der jeweils gegebenenfalls unabhängig voneinander ein- oder mehrfach mit Hydroxy, Nitro, Amino, Halogen, Alkoxy, Cyano, Hydroxycarbonyl. Alkoxycarbonyl, Alkylcarbamoyl, Cycloalkylcarbamoyl oder Phenyl substituierten Gruppierungen C₁-C₆-Alkyl, C₁-C₆-Alkyloxy, C₂-C₆-Alkenyl, C₂-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl, *N*-(C₁-C₆-Alkyl)amino, *N,N*-Di-(C₁-C₆-Alkyl)amino, oder *N*-(C₁-C₆-Alkylcarbonyl)amino steht; oder
Q für ein mit 0 - 4 Substituenten V substituierten Aryl oder für ein mit 0 - 4 Substituenten V substituierten 5 bzw. 6 gliedrigen Heteroaromaten steht, wobei
V unabhängig voneinander für Halogen, Cyano, Nitro, oder eine der jeweils gegebenenfalls unabhängig voneinander ein- oder mehrfach mit Hydroxy, Nitro, Amino, Halogen, Alkoxy, Cyano oder Phenyl substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di(C₁-C₆-Alkyl)amino steht;
R⁵ unabhängig voneinander für Wasserstoff, Halogen, Cyano, Nitro, Amino oder jeweils gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di-(C₁-C₆-Alkyl)amino steht;
Z¹ für ein jeweils gegebenenfalls mit Halogen substituiertes C₁-C₆-Alkyl steht;
Z² für Wasserstoff, Halogen, Cyano, Nitro, Amino oder ein jeweils gegebenenfalls mit Halogen substituiertes C₁-C₆-Alkyl, C₁-C₆-Alkylcarbonyl, C₁-C₆-Alkylsulfanyl, C₁-C₆-Alkylsulfinyl oder C₁-C₆-Alkylsulfonyl steht; und
Z³ für Wasserstoff oder ein jeweils gegebenenfalls mit Halogen substituiertes C₁-C₆-Alkyl, C₃-C₆-Cycloalkyl, C₂-C₄-Alkenyl, C₂-C₄-Alkinyl, Aryl oder Hetaryl steht.

3. Verbindung der Formel (I)gemäß Anspruch 1 oder 2, worin
R¹ für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
A₁ für CR² oder Stickstoff,
A₂ für Stickstoff,
A₃ für CR³ oder Stickstoff, und
A₄ für CR⁴ oder Stickstoff stehen,
wobei aber nicht mehr als drei der chemischen Gruppierungen A₁ bis A₄ gleichzeitig für Stickstoff stehen;
R² und R⁴ unabhängig voneinander für Wasserstoff, Methyl, Fluor und Chlor stehen; und
R³ für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
W für Sauerstoff oder Schwefel steht;
Q für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N*-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N*-Dimethylamino, *N,N-*Diethylamino steht; oder
Q für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
R⁵ unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
Z¹ für Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl steht;
Z² für Wasserstoff, Halogen, Cyano, Nitro,, Amino, Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl steht; und
Z³ für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl steht.

4. Verbindung der Formel (I) gemäß einem der Ansprüche 1 bis 3, worin
R¹ für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Methoxyethyl, Ethoxyethyl, Methoxymethyl, Ethoxymethyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
A₁ für CH,
A₂ für Stickstoff,
A₃ für CR³, und
A₄ für CH stehen;
R³ für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N*-Dimethylamino, *N*-Ethylamino, *N,N*-Diethylamino, Methylsulfonylamino, *N*-Methyl-methylsulfonylamino steht;
W für Sauerstoff steht; und
Q für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N*-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N*-Dimethylamino, *N,N-*Diethylamino steht; oder
Q für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
R⁵ für Wasserstoff, Methyl, Ethyl, 2-Methylethyl, tert.-Butyl, Fluor, Chlor, Brom, Iod, Nitro, Trifluormethyl, Amino steht;
Z¹ für Trifluormethyl oder Pentafluorethyl steht;
Z² für Trifluormethyl, Difluormethyl, Nitro, Methylsulfanyl, Methylsulfinyl, Methylsulfonyl, Fluor, Chlor, Brom, Cyano oder Iod steht; und
Z³ für Methyl, Ethyl, n-Propyl oder Wasserstoff steht.

5. Verbindung der Formel (I) gemäß einem der Ansprüche 1 bis 3, worin die Verbindung der Formel (I) eine Verbindung der Formel (Ia) darstellt 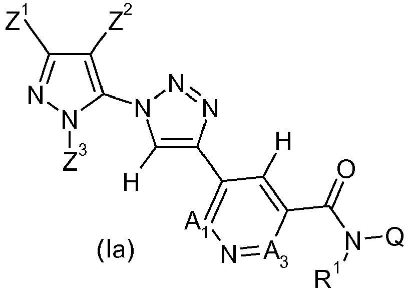 worin
R¹ für Wasserstoff, Methyl oder Ethyl, bevorzugt Wasserstoff, steht; und
A₁ für C-H oder C-(C₁-C₄-Alkyl) steht; bevorzugt für C-H steht; und
A₃ für CR³ steht; worin
R³ für Chlor, Fluor, Brom, Iod, C₁-C₄-Alkoxy oder Wasserstoff steht; bevorzugt für Chlor, Wasserstoff oder C₁-C₄-Alkoxy steht; mehr bevorzugt für Chlor, Wasserstoff oder Methoxy steht; und
Z¹ für C₁-C₄-Alkyl oder C₁-C₄-Halogenalkyl steht; bevorzugt für Trifluormethyl oder Pentafluorethyl steht; ganz besonders bevorzugt für Pentafluoroethyl steht; und
Z² für C₁-C₄-Halogenalkyl, Nitro, C₁-C₄-Alkylsulfanyl, C₁-C₄-Alkylsulfinyl, C₁-C₄-Alkylsulfonyl, Fluor, Chlor, Brom, Cyano oder Iod steht, bevorzugt für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
Z³ für Methyl, Ethyl, n-Propyl oder Wasserstoff steht; bevorzugt für Methyl steht; und
Q für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

6. Verbindung der Formel (Ia) gemäß Anspruch 5, worin die Verbindung der Formel (Ia) eine Verbindung der Formel (Ib) darstellt 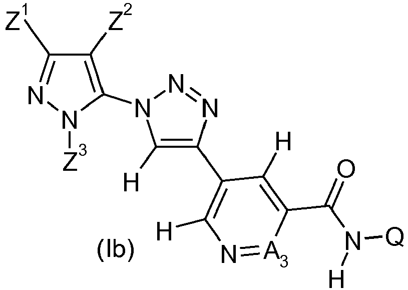 worin
A₃ für CR³ steht; worin
R³ für Chlor, Wasserstoff oder C₁-C₄-Alkoxy steht; mehr bevorzugt für Chlor, Wasserstoff oder Methoxy steht; und
Z¹ für Trifluormethyl oder Pentafluorethyl steht; ganz besonders bevorzugt für Pentafluoroethyl steht; und
Z² für Trifluormethyl, Difluormethyl, Nitro, Methylsulfanyl, Methylsulfinyl, Methylsulfonyl, Fluor, Chlor, Brom, Cyano oder Iod steht; ganz besonders bevorzugt für Trifluomethyl steht; und
Z³ für Methyl steht; und
Q für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl, C₁-C₄-Alkoxy C₁-C₄-Halogenalkoxy substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

7. Verbindung der Formel (Ib) gemäß Anspruch 6, worin
A₃ für CR³ steht; worin
R³ für Chlor, Wasserstoff oder C₁-C₄-Alkoxy steht; mehr bevorzugt für Chlor, Wasserstoff oder Methoxy steht; und
Z¹ für Pentafluoroethyl steht; und
Z² für Trifluomethyl steht; und
Z³ für Methyl steht; und
Q für Wasserstoff oder jeweils gegebenenfalls einfach, zweifach, dreifach oder vierfach unabhängig voneinander mit Cyano, Fluor, Chlor, Brom, Iod, Amino, Nitro, C₁-C₄-Alkyl oder C₁-C₄-Halogenalkyl substituiertes C₁-C₄-Alkyl, C₁-C₄-Hydroxyalkyl, C₁-C₄-Halogenalkyl, C₃-C₆-Cycloalkyl, Phenyl, Benzyl, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht.

8. Verbindung der Formel (Ib) gemäß Anspruch 6 oder 7, worin
A₃ für CR³ steht; worin
R³ für Chlor, Wasserstoff oder C₁-C₄-Alkoxy steht und
Z¹ für Pentafluoroethyl steht; und
Z² für Trifluomethyl steht; und
Z³ für Methyl steht; und
Q für Wasserstoff oder jeweils gegebenenfalls einfach, zweifach, dreifach oder vierfach unabhängig voneinander mit Cyano, Fluor, Chlor, Brom, Iod, substituiertes C₁-C₄-Alkyl, C₁-C₄-Halogenalkyl, C₃-C₆-Cycloalkyl, Phenyl, Benzyl, Pyridin, Pyrazol, Thiazol steht.

9. Verbindung der Formel (Ib) gemäß einem der Ansprüche 6 bis 8, worin
A₃ für C-R³ steht; und
R³ für Chlor, Wasserstoff oder C₁-C₄-Alkoxy steht; und
Z¹ für Pentafluoroethyl steht; und
Z² für Trifluomethyl steht; und
Z³ für Methyl steht; und
Q für jeweils gegebenenfalls einfach mit Cyano substituiertes oder jeweils gegebenenfalls einfach, zweifach, dreifach vierfach oder fünffach unabhängig voneinander mit Fluor, Chlor, Brom, Iod, substituiertes C₁-C₄-Alkyl oder C₃-C₆-Cycloalkyl steht.

10. Verbindung der allgemeinen Formel (I) gemäß einem der Ansprüche 1 bis 9 zur Verwendung zur Bekämpfung von Insekten, Spinnentieren und Nematoden.

11. Pharmazeutische Zusammensetzungen, enthaltend wenigstens eine Verbindung gemäß einem der Ansprüche 1 bis 9.

12. Verbindungen gemäß einem der Ansprüche 1 bis 9 zur Verwendung als Arzneimittel.

13. Verbindungen gemäß einem der Ansprüche 1 bis 9 zur Verwendung zur Bekämpfung von Parasiten auf Tieren.

14. Verwendung von Verbindungen gemäß einem der Ansprüche 1 bis 9 zum Schutz des Vermehrungsmaterials von Pflanzen, bevorzugt zum Schutz von Saatgut.

## Claims

1. Compound of the formula (I) 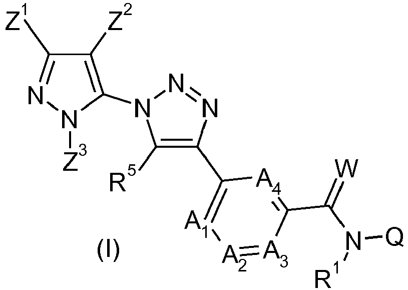 in which
R¹ represents hydrogen, in each case optionally substituted C₁-C₆-alkyl, C₂-C₆-alkenyl, C₂-C₆-alkynyl, C₃-C₇-cycloalkyl, C₃-C₇-cycloalkyl-C₁-C₆-alkyl, C₁-C₆-alkylcarbonyl, C₁-C₆-alkoxycarbonyl, C₁-C₆-alkoxy-C₁-C₆-alkyl, aryl-C₁-C₆-alkyl, heteroaryl-C₁-C₆-alkyl;
the chemical moieties
A₁ represents CR² or nitrogen,
A₂ represents nitrogen,
A₃ represents CR³ or nitrogen and
A₄ represents CR⁴ or nitrogen, but where not more than three of the chemical moieties A₁ to A₄ simultaneously represent nitrogen;
R², R³ and R⁴ independently of one another represent hydrogen, halogen, cyano, nitro, in each case optionally substituted C₁-C₆-alkyl, C₃-C₇-cycloalkyl, C₁-C₆-alkoxy, *N*-(C₁-C₆-alkoxy)imino-C₁-C₃-alkyl, C₁-C₆-alkylsulphanyl, C₁-C₆-alkylsulphinyl, C₁-C₆-alkylsulphonyl, *N-*(C₁-C₆-alkyl)amino, *N,N*-di(C₁-C₆-alkyl)amino, C₁-C₆-alkylsulphonylamino, or *N*-(C₁-C₆-alkyl)-C₁-C₆-alkylsulphonylamino;
W represents oxygen or sulphur;
Q represents hydrogen, formyl, hydroxy, amino or one of the in each case optionally substituted moieties C₁-C₆-alkyl, C₁-C₆-alkyloxy, C₂-C₆-alkenyl, C₂-C₆-alkynyl, C₃-C₇-cycloalkyl, hetero-C₁-C₆-cycloalkyl, C₃-C₆-cycloalkyl-C₁-C₆-alkyl, aryl-C₁-C₆-alkyl, heteroaryl-C₁-C₆-alkyl or represents a moiety *N*-(C₁-C₆-alkyl)amino, *N*-(C₁-C₆-alkylcarbonyl) amino, *N,N*-di(C₁-C₆-alkyl)amino; or
Q represents an unsaturated 6-membered carbocycle which is optionally polysubstituted by V or represents an unsaturated 5- or 6-membered heterocyclic ring which is optionally polysubstituted by V, where
V represents halogen, cyano, nitro, or one of the moieties C₁-C₆-alkyl, C₂-C₆-alkenyl, C₂-C₆-alkynyl, C₃-C₇-cycloalkyl, C₁-C₆-alkoxy, *N*-(C₁-C₆-alkoxy)imino-C₁-C₆-alkyl, C₁-C₆-alkylsulphanyl, C₁-C₆-alkylsulphinyl, C₁-C₆-alkylsulphonyl, *N*-(C₁-C₆-alkyl)amino or *N,N-*di(C₁-C₆-alkyl)amino, each of which is optionally substituted;
R⁵ independently of one another represent hydrogen, halogen, cyano, nitro, amino, in each case optionally substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, *N*-(C₁-C₆)-alkylamino or *N,N-*di(C₁-C₆-alkyl)amino;
Z¹ represents optionally substituted C₁-C₆-alkyl;
Z² represents hydrogen, halogen, cyano, nitro or in each case optionally substituted C₁-C₆-alkyl, C₁-C₆-alkylcarbonyl, C₁-C₆-alkylsulphanyl, C₁-C₆-alkylsulphinyl, C₁-C₆-alkylsulphonyl; and
Z³ represents hydrogen or in each case optionally substituted C₁-C₆-alkyl, C₃-C₇-cycloalkyl, C₃-C₆-alkenyl, C₃-C₆-alkynyl, aryl or hetaryl,
where "optionally substituted", unless any specific substituents are given, means that the corresponding group can be substituted one or more times by a substituent M¹, where, in the case of multiple substitutions, the substituents M1 can be identical or different and the substituents M1 are selected from the group consisting of: halogen, hydroxy, nitro, formyl, carboxy, cyano, amino, isocyano, azido, (C₁-C₄)-alkyl, (C₁-C₄)-haloalkyl, (C₂-C₄)-alkenyl, (C₂-C₄)-alkynyl, (C₃-C₆)-cycloalkyl, (C₁-C₄)-alkoxy, (C₁-C₄)-haloalkoxy, (C₁-C₄)-alkoxy-(C₁-C₄)-alkoxy, (C₁-C₄)-alkoxy-(C₁-C₆)-alkyl, *N*-(C₁-C₄)-alkoxyimino-(C₁-C₃)-alkyl, (C₁-C₄)-alkylsulphanyl, (C₁-C₄)-haloalkylsulphanyl, (C₁-C₄)-alkoxycarbonyl, (C₁-C₄)-alkylcarbonyl, carbamoyl, C₁-C₄-alkylcarbamoyl, C₃-C₇-cycloalkylcarbamoyl, mono- and *N,N*-di(C₁-C₄)-alkylaminocarbonyl, amino, (C₁-C₆)-acylamino, mono- and *N,N*-di(C₁-C₄)-alkylamino, tri(C₁-C₄)-alkylsilyl, (C₃-C₆)-cycloalkyl, C₆-aryl, heterocyclyl having 3 to 6 ring atoms, where each of the last-mentioned cyclic groups may also be attached via heteroatoms or a divalent functional CH₂ or C₂H₄ group, (C₁-C₄)-alkylsulphinyl, where both enantiomers of the (C₁-C₄)-alkylsulphinyl group are included, (C₁-C₄)-alkylsulphonyl, (C₁-C₄)-alkylphosphinyl, (C₁-C₄)-(C₁-C₄)-alkylsulphanyl-(C₁-C₄)-alkyl, (C₁-C₄)-alkoxy- (C₁-C₄)-alkyl, mono- and *N,N*-di(C₁-C₄)-alkylamino-(C₁-C₄)-alkyl and hydroxy(C₁-C₄)-alkyl, where the radicals M¹ can be unsubstituted or optionally, if they contain hydrocarbon-containing or nitrogen-hydrogen-containing fractions, can be substituted by one or more, preferably 1, 2 or 3 radicals M², where M² independently of the others is selected from the group consisting of amino, hydroxy, halogen, nitro, cyano, isocyano, mercapto, isothiocyanato, carboxy and carboamide.

2. Compound of the formula (I) according to Claim 1 in which
R¹ represents hydrogen, or C₁-C₆-alkyl, C₂-C₆-alkenyl, C₂-C₆-alkynyl, C₃-C₇-cycloalkyl, C₃-C₇-cycloalkyl-C₁-C₃-alkyl, C₁-C₆-alkylcarbonyl, C₁-C₆-alkoxycarbonyl, C₁-C₆-alkoxy-C₁-C₆-alkyl, aryl-(C₁-C₃)-alkyl, heteroaryl-(C₁-C₃)-alkyl, each of which is optionally mono- or polysubstituted independently of one another by halogen, cyano, alkoxy and alkoxycarbonyl;
the chemical moieties
A₁ represents CR² or nitrogen,
A₂ represents nitrogen,
A₃ represents CR³ or nitrogen, and
A₄ represents CR⁴ or nitrogen,
but where not more than three of the chemical moieties A₁ to A₄ simultaneously represent nitrogen;
R², R³ and R⁴ independently of one another represent hydrogen, halogen, cyano, nitro, in each case optionally substituted C₁-C₆-alkyl, C₃-C₇-cycloalkyl, C₁-C₆-alkoxy, *N*-(C₁-C₆-alkoxy)imino-C₁-C₃-alkyl, C₁-C₆-alkylsulphanyl, C₁-C₆-alkylsulphinyl, C₁-C₆-alkylsulphonyl, *N-*(C₁-C₆-alkyl)amino, *N,N*-di(C₁-C₆-alkyl)amino, C₁-C₆-alkylsulphonylamino, or *N*-(C₁-C₆-alkyl)-C₁-C₆-alkylsulphonylamino;
W represents oxygen or sulphur;
Q represents hydrogen, hydroxy, formyl or one of the moieties C₁-C₆-alkyl, C₁-C₆-alkyloxy, C₂-C₆-alkenyl, C₂-C₆-alkynyl, C₃-C₇-cycloalkyl, hetero-C₁-C₆-cycloalkyl, C₃-C₆-cycloalkyl-C₁-C₆-alkyl, aryl-C₁-C₆-alkyl, heteroaryl-C₁-C₆-alkyl, *N*-(C₁-C₆-alkyl)amino, *N,N*-di(C₁-C₆-alkyl)amino or *N*-(C₁-C₆-alkylcarbonyl)amino, each of which is optionally independently of the others mono- or polysubstituted by hydroxy, nitro, amino, halogen, alkoxy, cyano, hydroxycarbonyl, alkoxycarbonyl, alkylcarbamoyl, cycloalkylcarbamoyl or phenyl; or
Q represents aryl substituted by 0 - 4 substituents V or a 5- or 6-membered heteroaromatic substituted by 0 - 4 substituents V, where
V independently of one another represent halogen, cyano, nitro, or one of the moieties C₁-C₆-alkyl, C₂-C₆-alkenyl, C₂-C₆-alkynyl, C₃-C₇-cycloalkyl, C₁-C₆-alkoxy, *N*-(C₁-C₆-alkoxy)imino-C₁-C₆-alkyl, C₁-C₆-alkylsulphanyl, C₁-C₆-alkylsulphinyl, C₁-C₆-alkylsulphonyl, *N-*(C₁-C₆-alkyl)amino or *N,N*-di(C₁-C₆-alkyl)amino, each of which is optionally mono- or polysubstituted independently of the others by hydroxy, nitro, amino, halogen, alkoxy, cyano or phenyl;
R⁵ independently of one another represent hydrogen, halogen, cyano, nitro, amino, or in each case optionally halogen-substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, *N*-(C₁-C₆)-alkylamino or *N,N*-di(C₁-C₆-alkyl)amino;
Z¹ represents in each case optionally halogen-substituted C₁-C₆-alkyl;
Z² represents hydrogen, halogen, cyano, nitro, amino or an in each case optionally halogen-substituted C₁-C₆-alkyl, C₁-C₆-alkylcarbonyl, C₁-C₆-alkylsulphanyl, C₁-C₆-alkylsulphinyl or C₁-C₆-alkylsulphonyl, and
Z³ represents hydrogen or an in each case optionally halogen-substituted C₁-C₆-alkyl, C₃-C₆-cycloalkyl, C₂-C₄-alkenyl, C₂-C₄-alkynyl, aryl or hetaryl.

3. Compound of the formula (I) according to Claim 1 or 2 in which
R¹ represents hydrogen, methyl, ethyl, n-propyl, isopropyl, n-butyl, isobutyl, s-butyl, t-butyl, methoxymethyl, ethoxymethyl, propoxymethyl, 2-propyn-1-yl, 2-propen-1-yl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, methoxyethyl, ethoxyethyl, methoxymethyl, ethoxymethyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl;
the chemical moieties
A₁ represents CR² or nitrogen,
A₂ represents nitrogen,
A₃ represents CR³ or nitrogen, and
A₄ represents CR⁴ or nitrogen,
but where not more than three of the chemical moieties A₁ to A₄ simultaneously represent nitrogen;
R² and R⁴ independently of one another represent hydrogen, methyl, fluorine or chlorine; and
R³ represents hydrogen, fluorine, chlorine, bromine, iodine, cyano, nitro, methyl, ethyl, fluoromethyl, difluoromethyl, chlorodifluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, trifluoromethylsulphanyl, methylsulphonyl, methylsulphinyl, trifluoromethylsulphonyl, trifluoromethylsulphinyl, *N*-methylamino, *N,N-*dimethylamino, *N*-ethylamino, *N,N*-diethylamino, methylsulphonylamino, *N-*methylmethylsulphonylamino;
W represents oxygen or sulphur;
Q represents hydrogen, methyl, ethyl, n-propyl, 1-methylethyl, 1,1-dimethylethyl, 1-methylpropyl, n-butyl, 2-methylpropyl, 2-methylbutyl, hydroxymethyl, 2-hydroxypropyl, cyanomethyl, 2-cyanoethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, 1-cyanocyclopropyl, 1-methoxycarbonylcyclopropyl, 1-(*N-*methylcarbamoyl)cyclopropyl, 1-(*N-*cyclopropylcarbamoyl)cyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, prop-2-enyl, 2-methylprop-2-enyl, prop-2-ynyl, 1,1-dimethylbut-2-ynyl, 3-chloroprop-2-enyl, 3,3-dichloroprop-2-enyl, 3,3-dichloro-1,1-dimethylprop-2-enyl, phenyl, 2-chlorophenyl, 3-chlorophenyl, 4-chlorophenyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, 2-thienylmethyl, 3-thienylmethyl, benzyl, 2,6-difluorophenylmethyl, 3-fluorophenylmethyl, 2-fluorophenylmethyl, 2,5-difluorophenylmethyl, 1-phenylethyl, 4-chlorophenylethyl, 2-trifluoromethylphenylethyl, 1-pyridin-2-ylethyl, pyridin-2-ylmethyl, 5-fluoropyridin-2-ylmethyl, (6-chloropyridin-3-yl)methyl, pyrimidin-2-ylmethyl, methoxy, 2-ethoxyethyl, 2-(methylsulphanyl)ethyl, 1-methyl-2-(ethylsulphanyl)ethyl, 2-methyl-1-(methylsulphanyl)propan-2-yl, methoxycarbonyl, methoxycarbonylmethyl, NH₂, *N*-ethylamino, *N-*allylamino, *N,N*-dimethylamino, *N,N-*diethylamino; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0 - 4 substituents V, where
V independently of one another represent fluorine, chlorine, bromine, iodine, cyano, nitro, methyl, ethyl, isopropyl, difluoromethyl, trichloromethyl, chlorodifluoromethyl, dichlorofluoromethyl, trifluoromethyl, chloromethyl, bromomethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N*-methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, *N,N*-dimethylamino;
R⁵ independently of one another represent hydrogen, halogen, cyano, nitro, amino, methyl, ethyl, 1-methylethyl, tert-butyl, trifluoromethyl, difluoromethyl, methoxy, ethoxy, trifluoromethoxy, 2,2-difluoroethoxy, 2,2,2-trifluoroethoxy, methylcarbonyl, ethylcarbonyl, trifluoromethylcarbonyl, methylsulphanyl, methylsulphinyl, methylsulphonyl, trifluoromethylsulphonyl, trifluoromethylsulphanyl, trifluoromethylsulphinyl;
Z¹ represents methyl, ethyl, 1,1-dimethylethyl, difluoromethyl, trichloromethyl, chlorodifluoromethyl, dichlorofluoromethyl, trifluoromethyl, bromodichloromethyl, chloromethyl, bromomethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl;
Z² represents hydrogen, halogen, cyano, nitro, amino, methyl, ethyl, 1,1-dimethylethyl, difluoromethyl, trichloromethyl, chlorodifluoromethyl, dichlorofluoromethyl, trifluoromethyl, bromodichloromethyl, chloromethyl, bromomethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-t-butyl, heptafluoro-n-propyl, heptafluoroisopropyl, nonafluoro-n-butyl, methylsulphanyl, methylsulphinyl, methylsulphonyl, ethylthio, ethylsulphinyl, ethylsulphonyl, trifluoromethylsulphanyl, trifluoromethylsulphinyl, trifluoromethylsulphonyl, chlorodifluoromethylsulphanyl, chlorodifluoromethylsulphinyl, chlorodifluoromethylsulphonyl, dichlorofluoromethylsulphanyl, dichlorofluoromethylsulphinyl, dichlorofluoromethylsulphonyl; and
Z³ represents hydrogen, methyl, ethyl, n-propyl, isopropyl, n-butyl, isobutyl, s-butyl, t-butyl, ethenyl, 1-propenyl, 2-propenyl, 1-propynyl, 1-butynyl, difluoromethyl, trichloromethyl, chlorodifluoromethyl, dichlorofluoromethyl, trifluoromethyl, chloromethyl, bromomethyl, 1-fluoroethyl, 1-fluoro-1-methylethyl, 2-fluoroethyl, 2,2-difluoroethyl, 2,2,2-trifluoroethyl, phenyl, 2-chlorophenyl, 3-chlorophenyl, 4-chlorophenyl, 2,5-dichlorophenyl, 3,4-dichlorophenyl, 2,6-dichlorophenyl, 2,6-dichloro-4-trifluoromethylphenyl, 3-chloro-5-trifluoromethylpyridin-2-yl.

4. Compound of the formula (I) according to any of Claims 1 to 3 in which
R¹ represents hydrogen, methyl, ethyl, n-propyl, isopropyl, n-butyl, isobutyl, s-butyl, t-butyl, methoxymethyl, ethoxymethyl, propoxymethyl, 2-propyn-1-yl, 2-propen-1-yl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, methoxyethyl, ethoxyethyl, methoxymethyl, ethoxymethyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl;
the chemical moieties
A₁ represents CH,
A₂ represents nitrogen,
A₃ represents CR³, and
A₄ represents CH;
R³ represents hydrogen, fluorine, chlorine, bromine, iodine, cyano, nitro, methyl, ethyl, fluoromethyl, difluoromethyl, chlorodifluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, trifluoromethylsulphanyl, methylsulphonyl, methylsulphinyl, trifluoromethylsulphonyl, trifluoromethylsulphinyl, *N*-methylamino, *N,N-*dimethylamino, *N*-ethylamino, *N,N*-diethylamino, methylsulphonylamino, *N-*methylmethylsulphonylamino;
W represents oxygen; and
Q represents hydrogen, methyl, ethyl, n-propyl, 1-methylethyl, 1,1-dimethylethyl, 1-methylpropyl, n-butyl, 2-methylpropyl, 2-methylbutyl, hydroxymethyl, 2-hydroxypropyl, cyanomethyl, 2-cyanoethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, 1-cyanocyclopropyl, 1-methoxycarbonylcyclopropyl, 1-(*N-*methylcarbamoyl)cyclopropyl, 1-(*N-*cyclopropylcarbamoyl)cyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, prop-2-enyl, 2-methylprop-2-enyl, prop-2-ynyl, 1,1-dimethylbut-2-ynyl, 3-chloroprop-2-enyl, 3,3-dichloroprop-2-enyl, 3,3-dichloro-1,1-dimethylprop-2-enyl, phenyl, 2-chlorophenyl, 3-chlorophenyl, 4-chlorophenyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, 2-thienylmethyl, 3-thienylmethyl, benzyl, 2,6-difluorophenylmethyl, 3-fluorophenylmethyl, 2-fluorophenylmethyl, 2,5-difluorophenylmethyl, 1-phenylethyl, 4-chlorophenylethyl, 2-trifluoromethylphenylethyl, 1-pyridin-2-ylethyl, pyridin-2-ylmethyl, 5-fluoropyridin-2-ylmethyl, (6-chloropyridin-3-yl)methyl, pyrimidin-2-ylmethyl, methoxy, 2-ethoxyethyl, 2-(methylsulphanyl)ethyl, 1-methyl-2-(ethylsulphanyl)ethyl, 2-methyl-1-(methylsulphanyl)propan-2-yl, methoxycarbonyl, methoxycarbonylmethyl, NH₂, *N*-ethylamino, *N-*allylamino, *N,N*-dimethylamino, *N,N-*diethylamino; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0 - 4 substituents V, where
V independently of one another represent fluorine, chlorine, bromine, iodine, cyano, nitro, methyl, ethyl, isopropyl, difluoromethyl, trichloromethyl, chlorodifluoromethyl, dichlorofluoromethyl, trifluoromethyl, chloromethyl, bromomethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N*-methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, *N,N*-dimethylamino;
R⁵ represents hydrogen, methyl, ethyl, 2-methylethyl, tert-butyl, fluorine, chlorine, bromine, iodine, nitro, trifluoromethyl, amino;
Z¹ represents trifluoromethyl or pentafluoroethyl;
Z² represents trifluoromethyl, difluoromethyl, nitro, methylsulphanyl, methylsulphinyl, methylsulphonyl, fluorine, chlorine, bromine, cyano or iodine;
Z³ represents methyl, ethyl, n-propyl or hydrogen.

5. Compound of the formula (I) according to any of Claims 1 to 3 in which the compound of the formula (I) represents a compound of the formula (Ia) 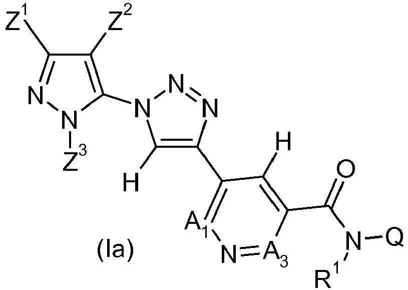 in which
R¹ represents hydrogen, methyl or ethyl, preferably hydrogen; and
A₁ represents C-H or C-(C₁-C₄-alkyl), preferably C-H; and
A₃ represents CR³; in which
R³ represents chlorine, fluorine, bromine, iodine, C₁-C₄-alkoxy or hydrogen, preferably chlorine, hydrogen or C₁-C₄-alkoxy, more preferably chlorine, hydrogen or methoxy; and
Z¹ represents C₁-C₄-alkyl or C₁-C₄-haloalkyl, preferably trifluoromethyl or pentafluoroethyl, very particularly preferably pentafluoroethyl; and
Z² represents C₁-C₄-haloalkyl, nitro, C₁-C₄-alkylsulphanyl, C₁-C₄-alkylsulphinyl, C₁-C₄-alkylsulphonyl, fluorine, chlorine, bromine, cyano or iodine, preferably trifluoromethyl, difluoromethyl, nitro, methylsulphanyl, methylsulphinyl, methylsulphonyl, fluorine, chlorine, bromine, cyano or iodine, very particularly preferably trifluoromethyl; and
Z³ represents methyl, ethyl, n-propyl or hydrogen, preferably methyl; and
Q represents hydrogen or C₁-C₄-alkyl, C₁-C₄-hydroxyalkyl, C₁-C₄-haloalkyl, C₃-C₆-cycloalkyl, phenyl, benzyl, triazine, pyridine, pyrazole, thiazole, isothiazole, oxazole, isoxazole, triazole, imidazole, furan, thiophene, pyrrole, oxadiazole, thiadiazole, each of which is optionally mono-, di-, tri- or tetrasubstituted independently of the others by cyano, fluorine, chlorine, bromine, iodine, amino, nitro, C₁-C₄-alkyl or C₁-C₄-haloalkyl.

6. Compound of the formula (Ia) according to Claim 5 in which the compound of the formula (Ia) represents a compound of the formula (Ib) 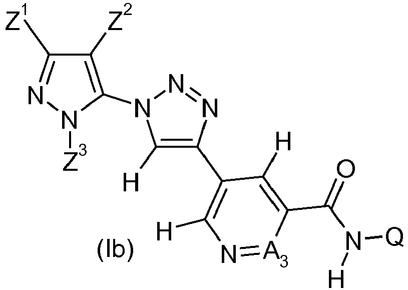 in which
A₃ represents CR³; in which
R³ represents chlorine, hydrogen or C₁-C₄-alkoxy, more preferably chlorine, hydrogen or methoxy; and
Z¹ represents trifluoromethyl or pentafluoroethyl, very particularly preferably pentafluoroethyl; and
Z² represents trifluoromethyl, difluoromethyl, nitro, methylsulphanyl, methylsulphinyl, methylsulphonyl, fluorine, chlorine, bromine, cyano or iodine, very particularly preferably trifluoromethyl;
Z³ represents methyl; and
Q represents hydrogen or C₁-C₄-alkyl, C₁-C₄-hydroxyalkyl, C₁-C₄-haloalkyl, C₃-C₆-cycloalkyl, phenyl, benzyl, triazine, pyridine, pyrazole, thiazole, isothiazole, oxazole, isoxazole, triazole, imidazole, furan, thiophene, pyrrole, oxadiazole, thiadiazole, each of which is optionally mono-, di-, tri- or tetrasubstituted independently of the others by cyano, fluorine, chlorine, bromine, iodine, amino, nitro, C₁-C₄-alkyl, C₁-C₄-haloalkyl, C₁-C₄-alkoxy or C₁-C₄-haloalkoxy.

7. Compound of the formula (Ib) according to Claim 6 in which
A₃ represents CR³; in which
R³ represents chlorine, hydrogen or C₁-C₄-alkoxy, more preferably chlorine, hydrogen or methoxy; and
Z¹ represents pentafluoroethyl; and
Z² represents trifluoromethyl; and
Z³ represents methyl; and
Q represents hydrogen or C₁-C₄-alkyl, C₁-C₄-hydroxyalkyl, C₁-C₄-haloalkyl, C₃-C₆-cycloalkyl, phenyl, benzyl, triazine, pyridine, pyrazole, thiazole, isothiazole, oxazole, isoxazole, triazole, imidazole, furan, thiophene, pyrrole, oxadiazole, thiadiazole, each of which is optionally mono-, di-, tri- or tetrasubstituted independently of the others by cyano, fluorine, chlorine, bromine, iodine, amino, nitro, C₁-C₄-alkyl or C₁-C₄-haloalkyl.

8. Compound of the formula (Ib) according to Claim 6 or 7 in which
A₃ represents CR³; in which
R³ represents chlorine, hydrogen or C₁-C₄-alkoxy and
Z¹ represents pentafluoroethyl; and
Z² represents trifluoromethyl; and
Z³ represents methyl; and
Q represents hydrogen or C₁-C₄-alkyl, C₁-C₄-haloalkyl, C₃-C₆-cycloalkyl, phenyl, benzyl, pyridine, pyrazole, thiazole, each of which is optionally mono-, di-, tri- or tetrasubstituted independently of the others by cyano, fluorine, chlorine, bromine, iodine.

9. Compound of the formula (Ib) according to any of Claims 6 to 8 in which
A₃ represents C-R³; and
R₃ represents chlorine, hydrogen or C₁-C₄-alkoxy; and
Z¹ represents pentafluoroethyl; and
Z² represents trifluoromethyl; and
Z³ represents methyl; and
Q represents C₁-C₄-alkyl or C₃-C₆-cycloalkyl, each of which is optionally monosubstituted by cyano or each of which is optionally mono-, di-, tri-, tetra- or pentasubstituted independently of the others by fluorine, chlorine, bromine, iodine.

10. Compound of the general formula (I) according to any of Claims 1 to 9 for use for controlling insects, arachnids and nematodes.

11. Pharmaceutical compositions comprising at least one compound according to any of Claims 1 to 9.

12. Compounds according to any of Claims 1 to 9 for use as medicaments.

13. Compounds according to any of Claims 1 to 9 for use for controlling parasites on animals.

14. Use of compounds according to any of Claims 1 to 9 for protecting the propagation material of plants, preferably for protecting seed.

## Revendications

1. Composé de formule (I) 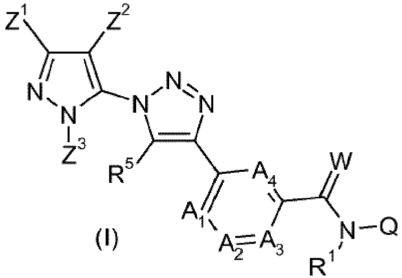 dans laquelle
R¹ représente hydrogène ; alkyle en C₁-C₆, alcényle en C₂-C₆, alcynyle en C₂-C₆, cycloalkyle en C₃-C₇, cycloalkyle en C₃-C₇-alkyle en C₁-C₆, alkylcarbonyle en C₁-C₆, alcoxycarbonyle en C₁-C₆, alcoxy en C₁-C₆-alkyle en C₁-C₆, aryl-alkyle en C₁-C₆, hétéroaryl-alkyle en C₁-C₆, chacun éventuellement substitués ;
les groupes chimiques ont les significations suivantes :
A₁ CR² ou azote,
A₂ azote,
A₃ CR³ ou azote et
A₄ CR⁴ ou azote,
pas plus de trois des groupes chimiques A₁ à A₄ ne représentant toutefois simultanément l'azote ;
R², R³ et R⁴ représentant indépendamment les uns des autres hydrogène, halogène, cyano, nitro ; alkyle en C₁-C₆, cycloalkyle en C₃-C₇, alcoxy en C₁-C₆, *N*-(alcoxy en C₁-C₆)-imino-alkyle en C₁-C₃, alkylsulfanyle en C₁-C₆, alkylsulfinyle en C₁-C₆, alkylsulfonyle en C₁-C₆, *N*-(alkyle en C₁-C₆) amino, *N,N*-di-(alkyle en C₁-C₆) amino, alkylsulfonylamino en C₁-C₆ ou *N*-(alkyle en C₁-C₆)-alkylsulfonylamino en C₁-C₆ ;
W représente oxygène ou soufre ;
Q représente hydrogène, formyle, hydroxy, amino ou
un des groupes chacun éventuellement substitués : alkyle en C₁-C₆, alkyloxy en C₁-C₆, alcényle en C₂-C₆, alcynyle en C₂-C₆, cycloalkyle en C₃-C₇, hétéro-cycloalkyle en C₁-C₆, cycloalkyle en C₃-C₆-alkyle en C₁-C₆, aryl-alkyle en C₁-C₆, hétéroaryl-alkyle en C₁-C₆ ou un groupe *N*-(alkyle en C₁-C₆)amino, *N*-(alkylcarbonyle en C₁-C₆) amino, *N,N*-di(alkyle en C₁-C₆) amino ; ou
Q représente un carbocycle insaturé à 6 chaînons éventuellement substitué à plusieurs reprises avec V, ou un cycle hétérocyclique insaturé à 5 ou 6 chaînons éventuellement substitué à plusieurs reprises avec V,
V représentant halogène, cyano, nitro ou un des groupes chacun éventuellement substitués : alkyle en C₁-C₆, alcényle en C₂-C₆, alcynyle en C₂-C₆, cycloalkyle en C₃-C₇, alcoxy en C₁-C₆, *N*-(alcoxy en C₁-C₆)-imino-alkyle en C₁-C₆, alkylsulfanyle en C₁-C₆, alkylsulfinyle en C₁-C₆, alkylsulfonyle en C₁-C₆, *N*-(alkyle en C₁-C₆) amino ou *N,N*-di(alkyle en C₁-C₆) amino ;
les R⁵ représentent indépendamment les uns des autres hydrogène, halogène, cyano, nitro, amino ; alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆, *N*-alkylamino en C₁-C₆ ou *N,N*-di-(alkyle en C₁-C₆)amino, chacun éventuellement substitués ;
Z¹ représente un alkyle en C₁-C₆ éventuellement substitué ;
Z² représente hydrogène, halogène, cyano, nitro ; ou alkyle en C₁-C₆, alkylcarbonyle en C₁-C₆, alkylsulfanyle en C₁-C₆, alkylsulfinyle en C₁-C₆, alkylsulfonyle en C₁-C₆, chacun éventuellement substitués ; et
Z³ représente hydrogène ; ou alkyle en C₁-C₆, cycloalkyle en C₃-C₇, alcényle en C₃-C₆, alcynyle en C₃-C₆, aryle ou hétaryle, chacun éventuellement substitués ;
« éventuellement substitué » signifiant, si aucun substituant spécifique n'est indiqué, que le groupe correspondant peut être substitué une fois ou plusieurs fois avec un substituant M¹ ; en cas de substitutions multiples, les substituants M¹ pouvant être identiques ou différents, et les substituants M¹ étant choisis dans le groupe constitué par : halogène, hydroxy, nitro, formyle, carboxy, cyano, amino, isocyano, azido, alkyle en (C₁-C₄), halogénoalkyle en (C₁-C₄), alcényle en (C₂-C₄), alcynyle en (C₂-C₄), cycloalkyle en (C₃-C₆), alcoxy en (C₁-C₄), halogénoalcoxy en (C₁-C₄), alcoxy en (C₁-C₄)-alcoxy en (C₁-C₄), alcoxy en (C₁-C₄)-alkyle en (C₁-C₆), *N*-alcoxy en (C₁-C₄)-imino-alkyle en (C₁-C₃), alkylsulfanyle en (C₁-C₄), halogénoalkylsulfanyle en (C₁-C₄), alcoxycarbonyle en (C₁-C₄), alkylcarbonyle en (C₁-C₄), carbamoyle, alkylcarbamoyle en (C₁-C₄), cycloalkylcarbamoyle en C₃-C₇, mono- et *N,N*-di-alkylaminocarbonyle en (C₁-C₄), amino, acylamino en (C₁-C₆), mono- et *N,N*-di-alkylamino en (C₁-C₄), tri-alkylsilyle en (C₁-C₄), cycloalkyle en (C₃-C₆), aryle en C₆, hétérocyclyle de 3 à 6 atomes de cycle, les groupes cycliques mentionnés en dernier pouvant chacun également être reliés par des hétéroatomes ou un groupe CH₂ ou C₂H₄ bivalent fonctionnel ; alkylsulfinyle en (C₁-C₄), les deux énantiomères du groupe alkylsulfinyle en (C₁-C₄) étant compris, alkylsulfonyle en (C₁-C₄), alkylphosphinyle en (C₁-C₄), alkylsulfanyle en (C₁-C₄)-alkyle en (C₁-C₄), alcoxy en (C₁-C₄)-alkyle en (C₁-C₄), mono- et *N,N*-di-alkyle en (C₁-C₄)-amino-alkyle en (C₁-C₄) et hydroxy-alkyle en (C₁-C₄), les radicaux M¹ pouvant être non substitués ou pouvant éventuellement, dans la mesure où ils contiennent des fractions hydrocarbonées ou contenant de l'azote et de l'hydrogène, être substitués avec un ou plusieurs, de préférence 1, 2 ou 3 radicaux M², les M² étant choisis indépendamment les uns des autres dans le groupe constitué par amino, hydroxy, halogène, nitro, cyano, isocyano, mercapto, isothiocyanato, carboxy et carboamide.

2. Composé de formule (I) selon la revendication 1, dans lequel
R¹ représente hydrogène ; ou alkyle en C₁-C₆, alcényle en C₂-C₆, alcynyle en C₂-C₆, cycloalkyle en C₃-C₇, cycloalkyle en C₃-C₇-alkyle en C₁-C₃, alkylcarbonyle en C₁-C₆, alcoxycarbonyle en C₁-C₆, alcoxy en C₁-C₆-alkyle en C₁-C₆, aryl-alkyle en C₁-C₃, hétéroaryl-alkyle en C₁-C₃, chacun éventuellement substitués une ou plusieurs fois indépendamment les uns des autres avec halogène, cyano, alcoxy et alcoxycarbonyle ;
les groupes chimiques ont les significations suivantes :
A₁ CR² ou azote,
A₂ azote,
A₃ CR³ ou azote et
A₄ CR⁴ ou azote,
pas plus de trois des groupes chimiques A₁ à A₄ ne représentant toutefois simultanément l'azote ;
R², R³ et R⁴ représentant indépendamment les uns des autres hydrogène, halogène, cyano, nitro ; alkyle en C₁-C₆, cycloalkyle en C₃-C₇, alcoxy en C₁-C₆, *N*-(alcoxy en C₁-C₆)-imino-alkyle en C₁-C₃, alkylsulfanyle en C₁-C₆, alkylsulfinyle en C₁-C₆, alkylsulfonyle en C₁-C₆, *N*-(alkyle en C₁-C₆) amino, *N,N*-di-(alkyle en C₁-C₆) amino, alkylsulfonylamino en C₁-C₆ ou *N*-(alkyle en C₁-C₆)-alkylsulfonylamino en C₁-C₆ ;
W représente oxygène ou soufre ;
Q représente hydrogène, hydroxy, formyle ; ou un des groupes chacun éventuellement substitués une ou plusieurs fois indépendamment les uns des autres avec hydroxy, nitro, amino, halogène, alcoxy, cyano, hydroxycarbonyle, alcoxycarbonyle, alkylcarbamoyle, cycloalkylcarbamoyle ou phényle : alkyle en C₁-C₆, alkyloxy en C₁-C₆, alcényle en C₂-C₆, alcynyle en C₂-C₆, cycloalkyle en C₃-C₇, hétéro-cycloalkyle en C₁-C₆, cycloalkyle en C₃-C₆-alkyle en C₁-C₆, aryl-alkyle en C₁-C₆, hétéroaryl-alkyle en C₁-C₆, *N*-(alkyle en C₁-C₆)amino, *N,N*-di(alkyle en C₁-C₆)amino ou *N-*(alkylcarbonyle en C₁-C₆)amino ; ou
Q représente un aryle substitué avec 0 à 4 substituants V ou un composé hétéroaromatique à 5 ou 6 chaînons substitué avec 0 à 4 substituants V,
les V représentant indépendamment les uns des autres halogène, cyano, nitro ou un des groupes à chaque fois éventuellement substitués une ou plusieurs fois indépendamment les uns des autres avec hydroxy, nitro, amino, halogène, alcoxy, cyano ou phényle : alkyle en C₁-C₆, alcényle en C₂-C₆, alcynyle en C₂-C₆, cycloalkyle en C₃-C₇, alcoxy en C₁-C₆, *N*-(alcoxy en C₁-C₆)-imino-alkyle en C₁-C₆, alkylsulfanyle en C₁-C₆, alkylsulfinyle en C₁-C₆, alkylsulfonyle en C₁-C₆, *N*-(alkyle en C₁-C₆)amino ou *N,N*-di(alkyle en C₁-C₆) amino ;
les R⁵ représentent indépendamment les uns des autres hydrogène, halogène, cyano, nitro, amino ; ou alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆, *N*-alkylamino en C₁-C₆ ou *N,N*-di-(alkyle en C₁-C₆)amino, chacun éventuellement substitués avec halogène ;
Z¹ représente un alkyle en C₁-C₆ à chaque fois éventuellement substitué avec halogène ;
Z² représente hydrogène, halogène, cyano, nitro, amino ; ou alkyle en C₁-C₆, alkylcarbonyle en C₁-C₆, alkylsulfanyle en C₁-C₆, alkylsulfinyle en C₁-C₆ ou alkylsulfonyle en C₁-C₆, chacun éventuellement substitués avec halogène ; et
Z³ représente hydrogène ; ou alkyle en C₁-C₆, cycloalkyle en C₃-C₆, alcényle en C₂-C₄, alcynyle en C₂-C₄, aryle ou hétaryle, chacun éventuellement substitués avec halogène.

3. Composé de formule (I) selon la revendication 1 ou 2, dans lequel
R¹ représente hydrogène, méthyle, éthyle, n-propyle, isopropyle, n-butyle, isobutyle, s-butyle, t-butyle, méthoxyméthyle, éthoxyméthyle, propoxyméthyle, 2-propin-1-yle, 2-propén-1-yle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, méthoxyéthyle, éthoxyéthyle, méthoxyméthyle, éthoxyméthyle, cyanométhyle, 2-cyanoéthyle, benzyle, 4-méthoxybenzyle, pyrid-2-yl-méthyle, pyrid-3-yl-méthyle, pyrid-4-yl-méthyle, 4-chloro-pyrid-3-yl-méthyle ;
les groupes chimiques ont les significations suivantes :
A₁ CR² ou azote,
A₂ azote,
A₃ CR³ ou azote et
A₄ CR⁴ ou azote,
pas plus de trois des groupes chimiques A₁ à A₄ ne représentant toutefois simultanément l'azote ;
R² et R⁴ représentant indépendamment l'un de l'autre hydrogène, méthyle, fluor et chlore ; et
R³ représentant hydrogène, fluor, chlore, brome, iode, cyano, nitro, méthyle, éthyle, fluorométhyle, difluorométhyle, chlorodifluorométhyle, trifluorométhyle, 2,2,2-trifluoroéthyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, *N*-méthoxyiminométhyle, 1-(*N-*méthoxyimino)-éthyle, méthylsulfanyle, trifluorométhylsulfanyle, méthylsulfonyle, méthylsulfinyle, trifluorométhylsulfonyle, trifluorométhylsulfinyle, *N*-méthylamino, *N,N-*diméthylamino, *N*-éthylamino, *N,N*-diéthylamino, méthylsulfonylamino, *N*-méthyl-méthylsulfonylamino ;
W représente oxygène ou soufre ;
Q représente hydrogène, méthyle, éthyle, n-propyle, 1-méthyléthyle, 1,1-diméthyléthyle, 1-méthylpropyle, n-butyle, 2-méthylpropyle, 2-méthylbutyle, hydroxyméthyle, 2-hydroxypropyle, cyanométhyle, 2-cyanoéthyle, 2-fluoroéthyle, 2,2-difluoroéthyle, 2,2,2-trifluoroéthyle, 1-(trifluorométhyl)éthyle, 2,2-difluoropropyle, 3,3,3-trifluropropyle, 2,2-diméthyl-3-fluoropropyle, cyclopropyle, 1-cyano-cyclopropyle, 1-méthoxycarbonyl-cyclopropyle, 1-(*N-*méthylcarbamoyl)cyclopropyle, 1-(*N-*cyclopropylcarbamoyl)-cyclopropyle, cyclopropyl-méthyle, cyclobutyle, cyclopentyle, cyclohexyle, 1-cyclopropyléthyle, bis(cyclopropyl)méthyle, 2,2-diméthylcyclopropyl-méthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, prop-2-ényle, 2-méthylprop-2-ényle, prop-2-inyle, 1,1-diméthylbut-2-inyle, 3-chloro-prop-2-ényle, 3,3-dichloro-prop-2-ényle, 3,3-dichloro-1,1-diméthylprop-2-ényle, phényle, 2-chlorophényle, 3-chlorophényle, 4-chlorophényle, oxétan-3-yle, thiétan-3-yle, 1-oxydo-thiétan-3-yle, 1,1-dioxydo-thiétan-3-yle, isoxazol-3-ylméthyle, 1,2,4-triazol-3-ylméthyle, 3-méthyloxétan-3-ylméthyle, 2-thiénylméthyle, 3-thiénylméthyle, benzyle, 2,6-difluorophénylméthyle, 3-fluorophénylméthyle, 2-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, 5-fluoropyridin-2-ylméthyle, (6-chloro-pyridin-3-yl)méthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, *N*-éthylamino, *N*-allylamino, *N,N*-diméthylamino, *N,N*-diéthylamino ; ou
Q représente un phényle, un naphtyle, une pyridazine, une pyrazine, une pyrimidine, une triazine, une pyridine, un pyrazole, un thiazole, un isothiazole, un oxazole, un isoxazole, un triazole, un imidazole, un furane, un thiophène, un pyrrole, un oxadiazole, un thiadiazole, substitué avec 0 à 4 substituants V ;
les V représentant indépendamment les uns des autres fluor, chlore, brome, iode, cyano, nitro, méthyle, éthyle, isopropyle, difluorométhyle, trichlorométhyle, chlorodifluorométhyle, dichlorofluorométhyle, trifluorométhyle, chlorométhyle, bromométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N*-méthoxyiminométhyle, 1-(*N-*méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthylamino ;
les R⁵ représentent indépendamment les uns des autres hydrogène, halogène, cyano, nitro, amino, méthyle, éthyle, 1-méthyléthyle, tert-butyle, trifluorométhyle, difluorométhyle, méthoxy, éthoxy, trifluorométhoxy, 2,2-difluoroéthoxy, 2,2,2-trifluoroéthoxy, méthylcarbonyle, éthylcarbonyle, trifluorométhylcarbonyle, méthylsulfanyle, méthylsulfinyle, méthylsulfonyle, trifluorométhylsulfonyle, trifluorométhylsulfanyle, trifluorméthylsulfinyle ;
Z¹ représente méthyle, éthyle, 1,1-diméthyléthyle, difluorométhyle, trichlorométhyle, chlorodifluorométhyle, dichlorofluorométhyle, trifluorométhyle, bromodichlorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-t-butyle, heptafluoro-n-propyle, heptafluoro-isopropyle, nonafluoro-n-butyle ;
Z² représente hydrogène, halogène, cyano, nitro, amino, méthyle, éthyle, 1,1-diméthyléthyle, difluorométhyle, trichlorométhyle, chlorodifluorométhyle, dichlorofluorométhyle, trifluorométhyle, bromodichlorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-t-butyle, heptafluoro-n-propyle, heptafluoro-isopropyle, nonafluoro-n-butyle, méthylsulfanyle, méthylsulfinyle, méthylsulfonyle, éthylthio, éthylsulfinyle, éthylsulfonyle, trifluorométhylsulfanyle, trifluorométhylsulfinyle, trifluorométhylsulfonyle, chloro-difluorométhylsulfanyle, chloro-difluorométhylsulfinyle, chloro-difluorométhylsulfonyle, dichloro-fluorométhylsulfanyle, dichloro-fluorométhylsulfinyle, dichloro-fluorométhylsulfonyle ; et
Z³ représente hydrogène, méthyle, éthyle, n-propyle, isopropyle, n-butyle, isobutyle, s-butyle, t-butyle, éthényle, 1-propényle, 2-propényle, 1-propinyle, 1-butinyle, difluorométhyle, trichlorométhyle, chlorodifluorométhyle, dichlorofluorométhyle, trifluorométhyle, chlorométhyle, bromométhyle, 1-fluoroéthyle, 1-fluoro-1-méthyléthyle, 2-fluoroéthyle, 2,2-difluoroéthyle, 2,2,2-trifluoroéthyle, phényle, 2-chlorophényle, 3-chlorophényle, 4-chlorophényle, 2,5-dichlorophényle, 3,4-dichlorophényle, 2,6-dichlorophényle, 2,6-dichloro-4-trifluorométhylphényle, 3-chloro-5-trifluorométhylpyridin-2-yle.

4. Composé de formule (I) selon l'une quelconque des revendications 1 à 3, dans lequel
R¹ représente hydrogène, méthyle, éthyle, n-propyle, isopropyle, n-butyle, i-butyle, s-butyle, t-butyle, méthoxyméthyle, éthoxyméthyle, propoxyméthyle, 2-propin-1-yle, 2-propén-1-yle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, méthoxyéthyle, éthoxyéthyle, méthoxyméthyle, éthoxyméthyle, cyanométhyle, 2-cyanoéthyle, benzyle, 4-méthoxybenzyle, pyrid-2-yl-méthyle, pyrid-3-yl-méthyle, pyrid-4-yl-méthyle, 4-chloro-pyrid-3-yl-méthyle ;
les groupes chimiques ont les significations suivantes :
A₁ CH,
A₂ azote,
A₃ CR³ et
A₄ CH,
R³ représente hydrogène, fluor, chlore, brome, iode, cyano, nitro, méthyle, éthyle, fluorométhyle, difluorométhyle, chlorodifluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N*-méthoxyiminométhyle, 1-(*N*-méthoxyimino)-éthyle, méthylsulfanyle, trifluorométhylsulfanyle, méthylsulfonyle, méthylsulfinyle, trifluorométhylsulfonyle, trifluorométhylsulfinyle, *N*-méthylamino, *N,N*-diméthylamino, *N*-éthylamino, *N,N-*diéthylamino, méthylsulfonylamino, N-méthyl-méthylsulfonylamino ;
W représente oxygène ; et
Q représente hydrogène, méthyle, éthyle, n-propyle, 1-méthyléthyle, 1,1-diméthyléthyle, 1-méthylpropyle, n-butyle, 2-méthylpropyle, 2-méthylbutyle, hydroxyméthyle, 2-hydroxypropyle, cyanométhyle, 2-cyanoéthyle, 2-fluoroéthyle, 2,2-difluoroéthyle, 2,2,2-trifluoroéthyle, 1-(trifluorométhyl)éthyle, 2,2-difluoropropyle, 3,3,3-trifluropropyle, 2,2-diméthyl-3-fluoropropyle, cyclopropyle, 1-cyano-cyclopropyle, 1-méthoxycarbonyl-cyclopropyle, 1-(*N-*méthylcarbamoyl)cyclopropyle, 1-(*N-*cyclopropylcarbamoyl)-cyclopropyle, cyclopropyl-méthyle, cyclobutyle, cyclopentyle, cyclohexyle, 1-cyclopropyléthyle, bis(cyclopropyl)méthyle, 2,2-diméthylcyclopropyl-méthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, prop-2-ényle, 2-méthylprop-2-ényle, prop-2-inyle, 1,1-diméthylbut-2-inyle, 3-chloro-prop-2-ényle, 3,3-dichloro-prop-2-ényle, 3,3-dichloro-1,1-diméthylprop-2-ényle, phényle, 2-chlorophényle, 3-chlorophényle, 4-chlorophényle, oxétan-3-yle, thiétan-3-yle, 1-oxydo-thiétan-3-yle, 1,1-dioxydo-thiétan-3-yle, isoxazol-3-ylméthyle, 1,2,4-triazol-3-ylméthyle, 3-méthyloxétan-3-ylméthyle, 2-thiénylméthyle, 3-thiénylméthyle, benzyle, 2,6-difluorophénylméthyle, 3-fluorophénylméthyle, 2-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, 5-fluoropyridin-2-ylméthyle, (6-chloro-pyridin-3-yl)méthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, *N*-éthylamino, *N*-allylamino, *N,N*-diméthylamino, *N,N*-diéthylamino ; ou
Q représente un phényle, un naphtyle, une pyridazine, une pyrazine, une pyrimidine, une triazine, une pyridine, un pyrazole, un thiazole, un isothiazole, un oxazole, un isoxazole, un triazole, un imidazole, un furane, un thiophène, un pyrrole, un oxadiazole, un thiadiazole, substitué avec 0 à 4 substituants V,
les V représentant indépendamment les uns des autres fluor, chlore, brome, iode, cyano, nitro, méthyle, éthyle, isopropyle, difluorométhyle, trichlorométhyle, chlorodifluorométhyle, dichlorofluorométhyle, trifluorométhyle, chlorométhyle, bromométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N*-méthoxyiminométhyle, 1-(*N-*méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthylamino ;
R⁵ représente hydrogène, méthyle, éthyle, 2-méthyléthyle, tert.-butyle, fluor, chlore, brome, iode, nitro, trifluorométhyle, amino ;
Z¹ représente trifluorométhyle ou pentafluoroéthyle ;
Z² représente trifluorométhyle, difluorométhyle, nitro, méthylsulfanyle, méthylsulfinyle, méthylsulfonyle, fluor, chlore, brome, cyano ou iode ; et
Z³ représente méthyle, éthyle, n-propyle ou hydrogène.

5. Composé de formule (I) selon l'une quelconque des revendications 1 à 3, dans lequel le composé de formule (I) est un composé de formule (Ia) 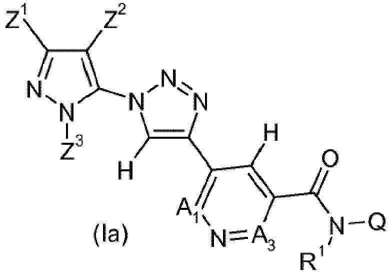 dans laquelle
R¹ représente hydrogène, méthyle ou éthyle, de préférence hydrogène ; et
A₁ représente C-H ou C-(alkyle en C₁-C₄); de préférence C-H ; et
A₃ représente CR³ ;
R³ représentant chlore, fluor, brome, iode, alcoxy en C₁-C₄ ou hydrogène ; de préférence chlore, hydrogène ou alcoxy en C₁-C₄ ; de manière davantage préférée chlore, hydrogène ou méthoxy ; et
Z¹ représente alkyle en C₁-C₄ ou halogénoalkyle en C₁-C₄ ; de préférence trifluorométhyle ou pentafluoroéthyle ; de manière tout particulièrement préférée pentafluoroéthyle ; et
Z² représente halogénoalkyle en C₁-C₄, nitro, alkylsulfanyle en C₁-C₄, alkylsulfinyle en C₁-C₄, alkylsulfonyle en C₁-C₄, fluor, chlore, brome, cyano ou iode, de préférence trifluorométhyle, difluorométhyle, nitro, méthylsulfanyle, méthylsulfinyle, méthylsulfonyle, fluor, chlore, brome, cyano ou iode ; de manière tout particulièrement préférée trifluorométhyle ; et
Z³ représente méthyle, éthyle, n-propyle ou hydrogène ; de préférence méthyle ; et
Q représente hydrogène ; ou alkyle en C₁-C₄, hydroxyalkyle en C₁-C₄, halogénoalkyle en C₁-C₄, cycloalkyle en C₃-C₆, phényle, benzyle, triazine, pyridine, pyrazole, thiazole, isothiazole, oxazole, isoxazole, triazole, imidazole, furane, thiophène, pyrrole, oxadiazole, thiadiazole, chacun éventuellement substitués une fois, deux fois, trois fois ou quatre fois indépendamment les uns des autres avec cyano, fluor, chlore, brome, iode, amino, nitro, alkyle en C₁-C₄ ou halogénoalkyle en C₁-C₄.

6. Composé de formule (Ia) selon la revendication 5, dans lequel le composé de formule (Ia) est un composé de formule (Ib) 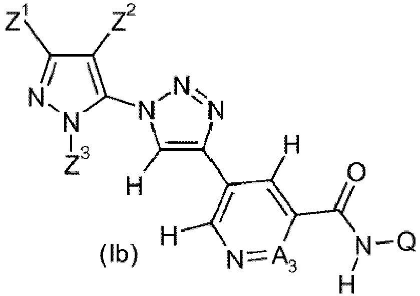 dans laquelle
A₃ représente CR³ ;
R³ représentant chlore, hydrogène ou alcoxy en C₁-C₄ ; de manière davantage préférée chlore, hydrogène ou méthoxy ; et
Z¹ représente trifluorométhyle ou pentafluoroéthyle ; de manière tout particulièrement préférée pentafluoroéthyle ; et
Z² représente trifluorométhyle, difluorométhyle, nitro, méthylsulfanyle, méthylsulfinyle, méthylsulfonyle, fluor, chlore, brome, cyano ou iode ; de manière tout particulièrement préférée trifluorométhyle ; et
Z³ représente méthyle ; et
Q représente hydrogène ; ou alkyle en C₁-C₄, hydroxyalkyle en C₁-C₄, halogénoalkyle en C₁-C₄, cycloalkyle en C₃-C₆, phényle, benzyle, triazine, pyridine, pyrazole, thiazole, isothiazole, oxazole, isoxazole, triazole, imidazole, furane, thiophène, pyrrole, oxadiazole, thiadiazole, chacun éventuellement substitués une fois, deux fois, trois fois ou quatre fois indépendamment les uns des autres avec cyano, fluor, chlore, brome, iode, amino, nitro, alkyle en C₁-C₄ ou halogénoalkyle en C₁-C₄.

7. Composé de formule (Ib) selon la revendication 6, dans lequel
A₃ représente CR³ ;
R³ représentant chlore, hydrogène ou alcoxy en C₁-C₄ ; de manière davantage préférée chlore, hydrogène ou méthoxy ; et
Z¹ représente pentafluoroéthyle ; et
Z² représente trifluorométhyle ; et
Z³ représente méthyle ; et
Q représente hydrogène ; ou alkyle en C₁-C₄, hydroxyalkyle en C₁-C₄, halogénoalkyle en C₁-C₄, cycloalkyle en C₃-C₆, phényle, benzyle, triazine, pyridine, pyrazole, thiazole, isothiazole, oxazole, isoxazole, triazole, imidazole, furane, thiophène, pyrrole, oxadiazole, thiadiazole, chacun éventuellement substitués une fois, deux fois, trois fois ou quatre fois indépendamment les uns des autres avec cyano, fluor, chlore, brome, iode, amino, nitro, alkyle en C₁-C₄ ou halogénoalkyle en C₁-C₄.

8. Composé de formule (Ib) selon la revendication 6 ou 7, dans lequel
A₃ représente CR³ ;
R³ représentant chlore, hydrogène ou alcoxy en C₁-C₄ ; et
Z¹ représente pentafluoroéthyle ; et
Z² représente trifluorométhyle ; et
Z³ représente méthyle ; et
Q représente hydrogène ; ou alkyle en C₁-C₄, halogénoalkyle en C₁-C₄, cycloalkyle en C₃-C₆, phényle, benzyle, pyridine, pyrazole, thiazole, chacun éventuellement substitués une fois, deux fois, trois fois ou quatre fois indépendamment les uns des autres avec cyano, fluor, chlore, brome, iode.

9. Composé de formule (Ib) selon l'une quelconque des revendications 6 à 8, dans lequel
A₃ représente C-R³ ;
R³ représentant chlore, hydrogène ou alcoxy en C₁-C₄ ; et
Z¹ représente pentafluoroéthyle ; et
Z² représente trifluorométhyle ; et
Z³ représente méthyle ; et
Q représente alkyle en C₁-C₄ ou cycloalkyle en C₃-C₆, chacun éventuellement substitués une fois avec cyano ou chacun éventuellement substitués une fois, deux fois, trois fois, quatre fois ou cinq fois indépendamment les uns des autres avec fluor, chlore, brome, iode.

10. Composé de formule générale (I) selon l'une quelconque des revendications 1 à 9, destiné à une utilisation pour lutter contre des insectes, des araignées et des nématodes.

11. Compositions pharmaceutiques, contenant au moins un composé selon l'une quelconque des revendications 1 à 9.

12. Composés selon l'une quelconque des revendications 1 à 9, destinés à une utilisation en tant que médicament.

13. Composés selon l'une quelconque des revendications 1 à 9, destinés à une utilisation pour lutter contre des parasites sur des animaux.

14. Utilisation de composés selon l'une quelconque des revendications 1 à 9 pour la protection du matériau reproductif de plantes, de préférence pour la protection de graines.
